# EUROPEAN PATENT APPLICATION

(11) **EP 4 660 319 A1**
(43) Date of publication of application: **10.12.2025**
(21) Application number: 25174588.1
(22) Date of filing: 06.05.2025
(51) Int. Cl.: C12Q 1/6869

(54) **METHOD FOR SEQUENCING OF A DNA STRAND**

(30) Priority: 14.05.2024 US 202418663817
(71) Applicant: Robert Bosch GmbH, 70442 Stuttgart (DE)
(72) Inventor: Grumaz, Christian, 71336 Waiblingen (DE); Pilsl, Sebastian, 70191 Stuttgart (DE); Fomina, Nadezda, Redwood City, California, 94061 (US); Baechtle, Sinja Theres, 79110 Freiburg (DE)

(57) **Abstract**

The disclosure relates to a method for sequencing of a DNA strand, the method includes (S200) providing a double-stranded DNA fragment with a first strand and a second strand, the DNA strand to be sequenced corresponding to the first strand or the second strand, (S300A) connecting the first and second strands with a first hairpin oligonucleotide and a second hairpin oligonucleotide, (S500A) amplifying the ligated product obtained in step (S300A) using rolling circle amplification (RCA), (S600A) amplifying the ligated product obtained in step (S300A) using RCA or the amplicons obtained in step (S500A) using primer extension using a first group of redox-modified nucleotides and a second group of redox-modified nucleotides, and (S800A) determining the sequence of the DNA strand by sequencing the amplicons (80) obtained in step (S600A) using an electrochemical sequencing method.

## Description

### TECHNICAL FIELD

The disclosure relates to methods for the sequencing of a DNA strand.

### BACKGROUND

Sequencing of nucleic acids such as DNA and RNA is of great importance in research and medicine. Several methods are known for the sequencing of nucleic acids. These include third generation sequencing methods with which a single nucleic acid molecule can be sequenced. For example, nucleotides are used with different fluorescence markings, which are optically detected in real time during their installation in a complementary nucleic acid strand. A further variant of the third-generation sequencing is the nanopore sequencing where nucleic acid is maintained by a nanopore, and the nucleotide of the row is identified by the pore following changes in ion current.

WO 2019/086305 A1 relates to a method for electro-chemical sequencing of DNA and a suitable device for this purpose. To do this, nucleotides with different redox species are incorporated into the DNA-strand to be sequenced. The DNA-beam modified in this way will then be maintained through at least one marginal electrode through which the nucleotides of the range are oxidized or reduced. The oxidation or reduction produces an electrochemical signal against which nucleotides are identified. The method is also known as sequencing by electronic tunneling, short SBET (from the English "sequencing by electronic tunneling").

Methods for the sequencing of nucleic acids usually begin with preparation of the nucleic acid for sequencing, in particular a nucleic acid library for sequencing. For the production of a nucleic acid library, it is known to fragment the nucleic acids, repair the ends of the fragments, and connect the fragments with adapters.

WO 2021/236792 A1 relates to methods for production of nucleic acid libraries for sequencing, where adaptors are added to the ends of double-stranded DNA fragments. As an adaptor, Y-shaped adapters containing clear molecular identification sequences (UMI sequences) are open. The amplification of the design is followed by a polymerase chain reaction.

WO 2005/093094 A2 relates to methods for sequencing of nucleic acids, where hairpin adaptors are attached to the ends of double-stranded DNA fragments. This creates a circular DNA. It follows a Rolling Circle amplification of circular DNA.

WO 2009/120372 A2 relates to addition of hairpin adapters to the ends of a double-stranded DNA fragment, resulting in circular DNA. Here, too, Rolling Circle amplification is followed by circular DNA.

### SUMMARY OF THE INVENTION

According to an embodiment, the method for sequencing of a DNA strand is disclosed. The method may include (S200) providing a double-stranded DNA fragment with a first strand having a 5' end and a 3' end and a second strand having a 5' end and a 3' end, the first and the second strands being complementary to each other, the DNA strand to be sequenced corresponding to the first strand or the second strand; (S300A) forming a ligated product by connecting: the 5' end of the first strand to a 3' end of a first hairpin oligonucleotide, the 3' end of the second strand to a 5' end of the first hairpin oligonucleotide, the 3' end of the first strand to a 5' end of a second hairpin oligonucleotide, and the 5' end of the second strand to a 3' end of the second hairpin oligonucleotide, the first hairpin oligonucleotide and the second hairpin oligonucleotide each providing a single-stranded section of the ligated product; (S500A) amplifying the ligated product obtained in step (S300A) by rolling circle amplification (RCA) to produce amplicons of the ligated product; (S600A) amplifying the ligated product obtained in step (S300A) or the amplicons of the ligated product obtained in step (S500A) by primer extension using a first group of redox-modified nucleotides and a second group of redox-modified nucleotides, each redox-modified nucleotide in the first group having a first redox species with a first oxidation or reduction potential and each redox-modified nucleotide in the second group having a second redox species with a second oxidation or reduction potential, the first group consisting of deoxyadenosine triphosphate (dATP), deoxythymidine triphosphate (dTTP), or deoxyuridine triphosphate (dUTP), the second group consisting of deoxycytidine triphosphate (dCTP) or deoxyguanosine triphosphate (dGTP); and

(S800A) determining the sequence of the DNA strand by sequencing the amplicons obtained in step (S600A) by electrochemical sequencing. The method may also include (S400A) selecting a correctly ligated product obtained in step (S300A) by an exonuclease treatment. The method may also include (S700A) selecting only the amplicons having the redox modified nucleotides obtained in the step (S600A) for the step (S800A). The (S700B) selecting may be by streptavidine purification. The method may also include (S250) forming overhang sequences in the double-stranded DNA fragment by dA-tailing of the first and second strands and (S260A) forming overhang sequences by dT-tailing of the first and second hairpin oligonucleotides. The method may also include (S100) fragmenting the double-stranded DNA into double-stranded DNA fragments with the first strand having the 5' end and a 3' end and the second strand having the 5' end and a 3' end, and (S150) performing DNA end repair of the DNA fragments obtained in step (S100).

In another embodiment, a method for sequencing of a DNA strand is disclosed. The method may include (S200) providing a double-stranded DNA fragment with a first strand having a 5' end and a 3' end and a second strand having a 5' end and a 3' end, the first and the second strands being complementary to each other, the DNA strand to be sequenced corresponding to the first strand or the second strand; (S300B) forming a ligated product by connecting: the 5' end of the first strand and the 3' end of the second strand to a first UMI oligonucleotide, and the 3' end of the first strand and the 5' end of the second strand to a second UMI oligonucleotide, the first UMI oligonucleotide and the second UMI oligonucleotide each being two-stranded, Y-shaped oligonucleotides having a paired end portion and an unpaired end portion, the paired end portions being connected to the first strand and the second strand, respectively, the paired end portion on each strand having a unique molecular identification sequence (UMI sequence) arranged terminally, the UMI sequence of the first UMI oligonucleotide being different from the UMI sequence of the second UMI oligonucleotide, the first strand being connected to the UMI sequence of the first UMI oligonucleotide and the second strand being connected to the UMI sequence of the second UMI oligonucleotide; (S500B) amplifying the ligated product obtained in step (S300B) using polymerase chain reaction (PCR) to generate amplicons; (S600B) amplifying the amplicons obtained in step (S500B) by primer extension using a first group of redox-modified nucleotides and a second group of redox-modified nucleotides, each redox-modified nucleotide in the first group having a first redox species with a first oxidation or reduction potential and each redox-modified nucleotide in the second group having a second redox species with a second oxidation or reduction potential, the first group consisting of deoxyadenosine triphosphate (dATP), deoxythymidine triphosphate (dTTP), or deoxyuridine triphosphate (dUTP), the second group consisting of deoxycytidine triphosphate (dCTP) or deoxyguanosine triphosphate (dGTP); and (S800B) determining the sequence of the DNA strand by sequencing the amplicons obtained in step (S600B) by electrochemical sequencing. The method may also include (S700B) selecting only the amplicons having the redox modified nucleotides for the step (S800B). The (S700B) selecting further comprises removing DNA molecules lacking the redox modified nucleotides. The method may also include (S250) forming overhang sequences in the double-stranded DNA fragment by dA-tailing of the first and second strands and (S260B) forming overhang sequences by dT-tailing of the first UMI oligonucleotide and the second UMI oligonucleotide. The method may also include (S100) fragmenting the double-stranded DNA into double-stranded DNA fragments with the first strand having the 5' end and a 3' end and the second strand having the 5' end and a 3' end, and (S150) performing DNA end repair of the DNA fragments obtained in step (S100).

In yet another embodiment, a method for sequencing of a DNA strand is disclosed. The method may include (S200) providing a double-stranded DNA fragment with a first strand having a 5' end and a 3' end and a second strand having a 5' end and a 3' end, the first and the second strands being complementary to each other, the DNA strand to be sequenced corresponding to the first strand or the second strand; (S300C) forming a ligated product by connecting: the 5' end of the first strand and the 3' end of the second strand to a first oligonucleotide, and the 3' end of the first strand and the 5' end of the second strand to a second oligonucleotide, one of the first and second oligonucleotides being a hairpin oligonucleotide and the other of the first and second oligonucleotides being a Y-shaped oligonucleotide; the hairpin oligonucleotide providing a single-stranded portion of the ligated product, the Y-shaped oligonucleotide being a two-stranded oligonucleotide with a paired end portion and an unpaired end portion, the paired end portion being connected to the first strand and the second strand, (S600C) forming amplicons by amplifying the ligated product obtained in step (S300C) by primer extension using a first group of redox-modified nucleotides and a second group of redox-modified nucleotides, each redox-modified nucleotide in the first group having a first redox species with a first oxidation or reduction potential and each redox-modified nucleotide in the second group having a second redox species with a second oxidation or reduction potential, the first group consisting of deoxyadenosine triphosphate (dATP), deoxythymidine triphosphate (dTTP), or deoxyuridine triphosphate (dUTP), the second group consisting of deoxycytidine triphosphate (dCTP) or deoxyguanosine triphosphate (dGTP); and (S800C) determining the sequence of the DNA strand by sequencing the amplicons obtained in step (S600C) by electrochemical sequencing. The paired end portion of the Y-shaped oligonucleotide on one strand may have a UMI sequence arranged terminally. The hairpin oligonucleotide may have a UMI sequence arranged terminally. The method may also include (S700C) selecting only the amplicons having the redox modified nucleotides obtained in step (S600C) for the step (S800C). The method may also include (S700C) selecting is by streptavidine purification. The method may also include (S400C) selecting a correctly ligated product obtained in step (S300A) by a first capture oligonucleotide binding to a specific unique portion of the first oligonucleotide and by a second capture oligonucleotide binding to a specific unique portion of the second oligonucleotide. The method may also include (S450C) amplifying the ligated product selected in step (S400C) using polymerase chain reaction (PCR). The method may also include (S250) forming overhang sequences in the double-stranded DNA fragment by dA-tailing of the first and second strands and (S260C) forming overhang sequences by dT-tailing of the first oligonucleotide and the second oligonucleotide. The method may also include (S100) fragmenting the double-stranded DNA into double-stranded DNA fragments with the first strand having the 5' end and a 3' end and the second strand having the 5' end and a 3' end, and (S150) performing DNA end repair of the DNA fragments obtained in step (S100).

### BRIEF DESCRIPTION OF THE DRAWINGS

Figures 1A-D are non-limiting examples of redox modified nucleotides; specifically
Fig. 1A is a redox modified nucleotide of the species dATP, where the redox species is anthraquinone;
Fig. 1B is a redox modified nucleotide of the kind of dUTP, where the redox species is methylene blue;
Fig. 1C a redox modified nucleotide of the species dGTP, where the redox species is ferrocene;
Fig. 1D a redox modified nucleotide of the species dCTP, where the redox species is phenothiazine;
Figures 2A and 2B are additional non-limiting examples of redox modified nucleotides; specifically

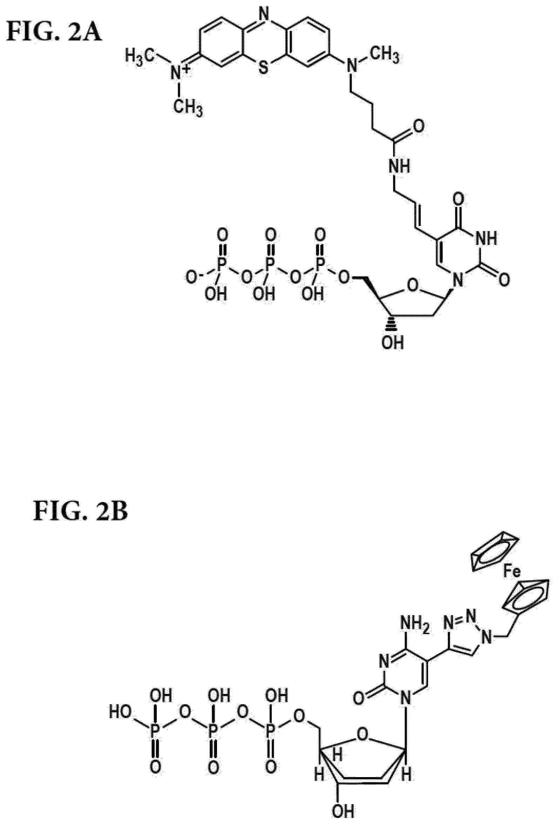

Fig. 2A is a redox-modified nucleotide of the type dUTP, where the redox species is the methylene blue derivative ATTO MB2 and the redox-modified nucleotide is also referred to as aminoallyl-dUTP-ATTO-MB2 or MB-dUTP;
Fig. 2B is a redox-modified nucleotide of the type dCTP, where the redox species is the ferrocene derivative FcN-C2 and the redox-modified nucleotide is also referred to as FcN-C2-dCTP;

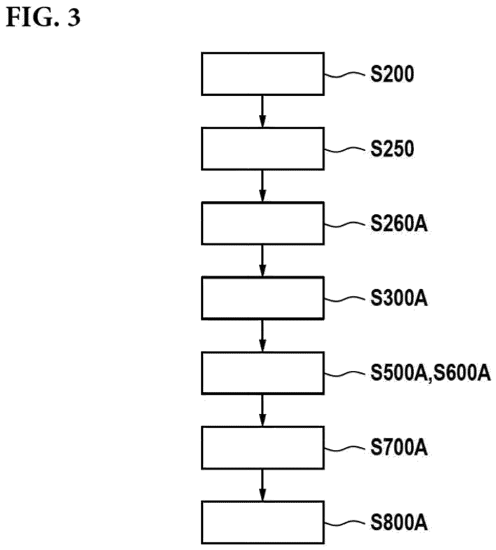

Figure 3 is a schematic presentation of a first method for DNA-strand sequencing ("Rolling Circle Method");

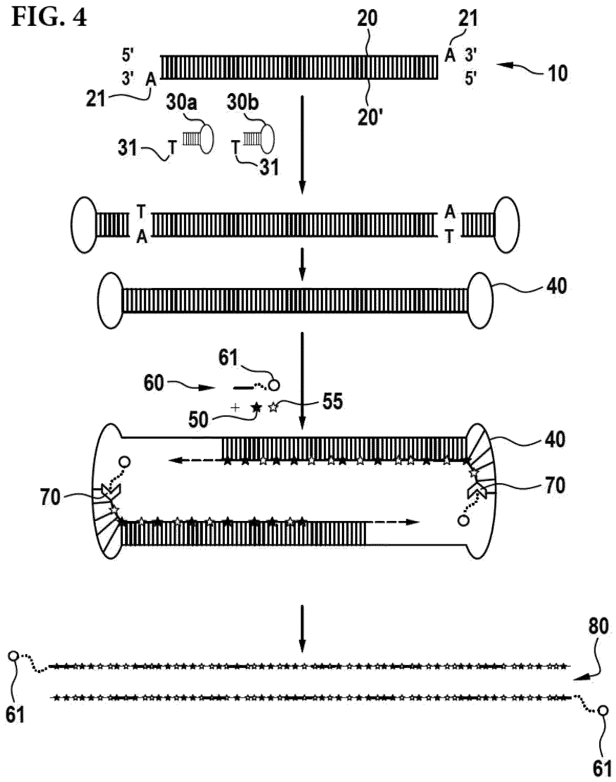

Figure 4 is a schematic presentation of the Rolling Circle Method shown in Fig. 3 including steps to prepare DNA fragments for sequencing;

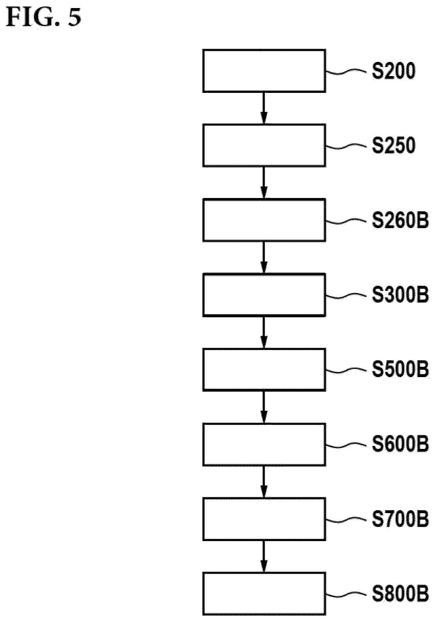

Figure 5 is a schematic presentation of a second method for DNA-strand sequencing ("UMI Method");

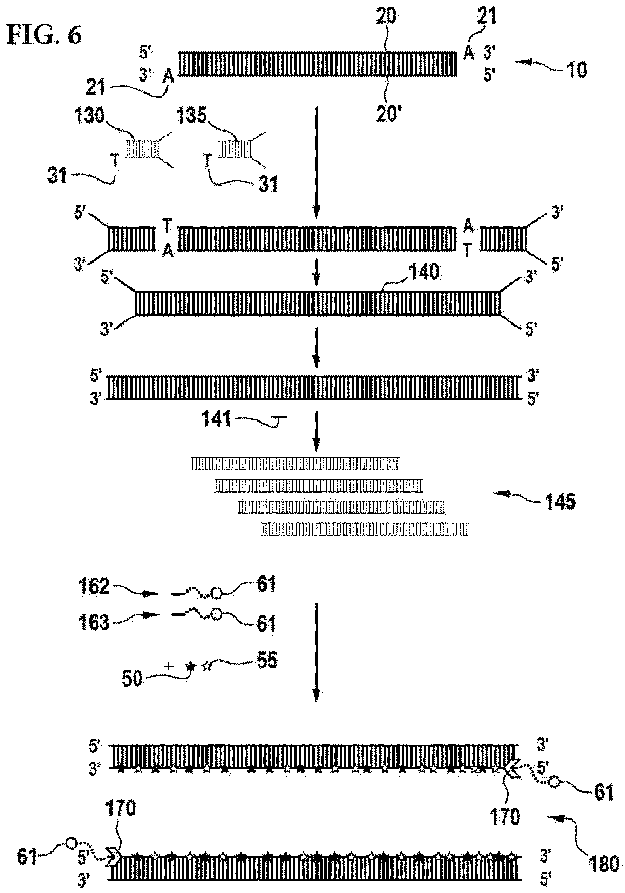

Figure 6 is a schematic presentation of the UMI Method shown in Fig. 5, part of which relates to the preparation of DNA fragments for sequencing;

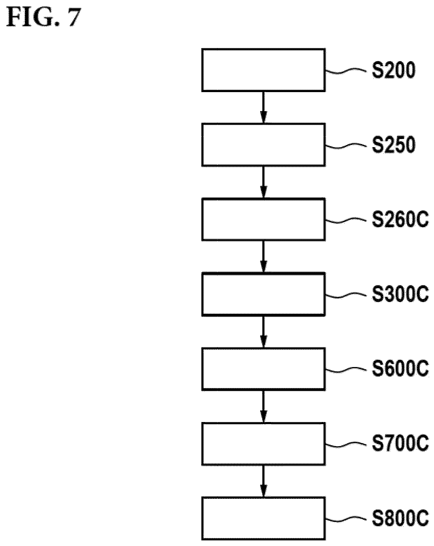

Figure 7 is schematic presentation of a third method for DNA-strand sequencing ("2D Method");

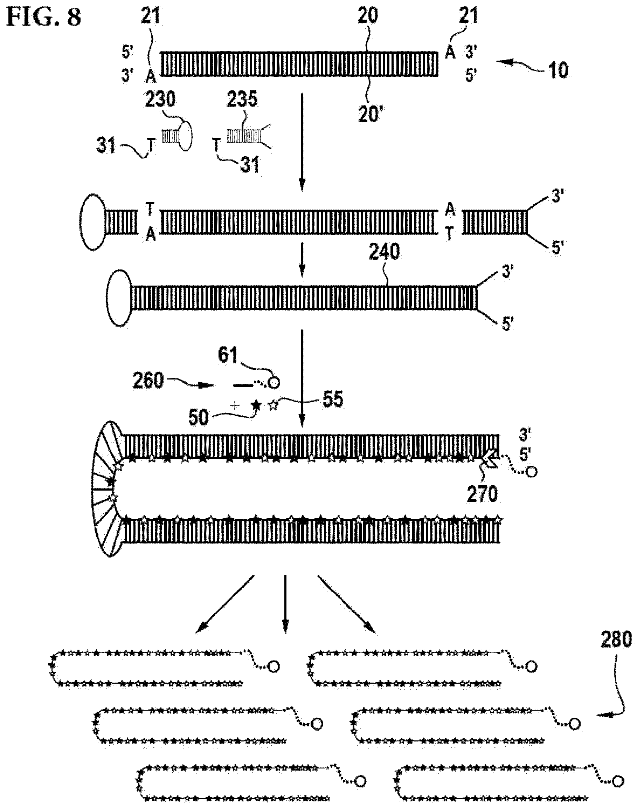

Figure 8 is a schematic presentation of the 2D Method shown in Fig. 7, part of which relates to the preparation of DNA fragments for sequencing;

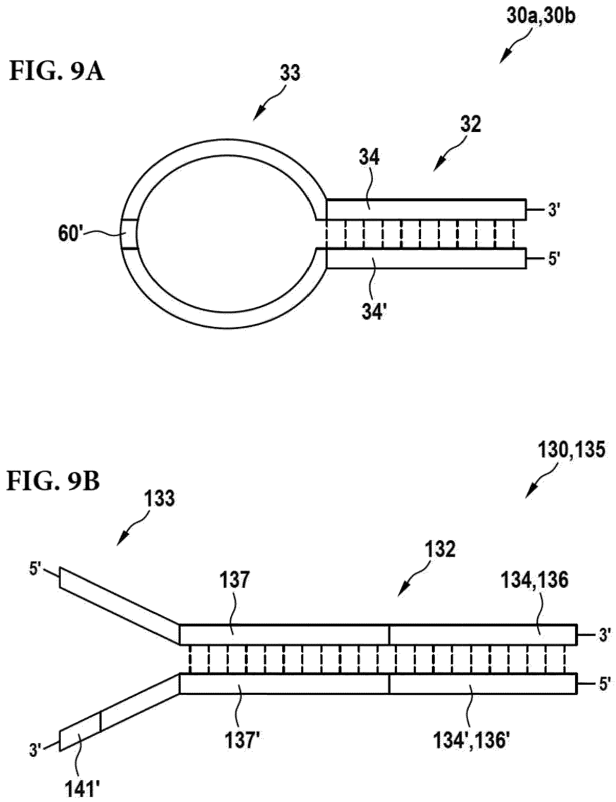

Figure 9A is a schematic representation of the first and second hairpin oligonucleotide of the Rolling Circle Method;
Figure 9B is a schematic representation of the first and the second UMI oligonucleotide of the UMI method; and

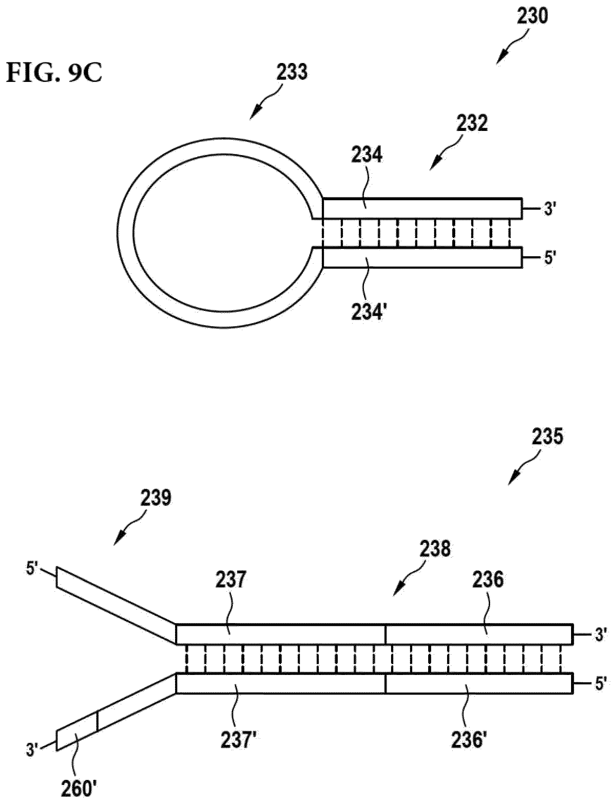

Figure 9C is a schematic representation of the first and second oligonucleotide of the 2D method.

### DETAILED DESCRIPTION

There is a need to provide alternative methods for nucleic acid sequencing.

The object of the disclosure is achieved by a first method for the sequencing of a DNA strand, a second method for sequencing of a DNA strand, and a third method for sequencing of a DNA strand.

The first method for the sequencing of a DNA strand includes:
(S200) providing a double-stranded DNA fragment with a first strand which has a 5' end and a 3' end, and a second strand, which has a 5' end and a 3' end, wherein the first strand and the second strand are complementary to each other, the DNA strand to be sequenced corresponding to the first strand or the second strand,
(S300A) connecting the 5' end of the first strand to a 3' end of a first hairpin oligonucleotide and connecting the 3' end of the second strand to a 5' end of the first hairpin oligonucleotide, and connecting the 3' end of the first strand to a 5' end of a second hairpin oligonucleotide and connecting the 5' end of the second strand to a 3' end of the second hairpin oligonucleotide, wherein the first hairpin oligonucleotide and the second hairpin oligonucleotide each provide a single-stranded portion of the resulting ligated product,
(S500A) amplifying the combined product obtained in step (S300A) using rolling circle amplification (RCA),
(S600A) amplifying the ligated product obtained in step (S300A) by RCA or the amplicons obtained in step (S500A) by primer extension using a first group of redox-modified nucleotides and a second group of redox-modified nucleotides, wherein each redox-modified nucleotide in the first group has a first redox species with a first oxidation or reduction potential and each redox-modified nucleotide in the second group has a second redox species with a second oxidation or reduction potential, wherein the first group includes, comprises, or consists of redox-modified nucleotides of the type deoxyadenosine triphosphate (dATP) or of redox-modified nucleotides of the type deoxythymidine triphosphate (dTTP) or of redox-modified nucleotides of the type deoxyuridine triphosphate (dUTP), and the second group includes, comprises, or consists of redox-modified nucleotides of the type deoxycytidine triphosphate (dCTP) or of redox-modified nucleotides of the type deoxyguanosine triphosphate (dGTP), and
(S800A) determining the sequence of DNA strand by sequencing the amplicons obtained in step (S600A) by an electrochemical sequencing method.

The first method for the sequencing of a DNA strand is also referred to as the "Rolling Circle Method."

The method may be used to sequence a DNA stand, *i.e.* to determine a nucleotide sequence of the DNA strand. The DNA strand is also referred to as the initial DNA strand. The DNA strand corresponds to the first strand or second strand of double-stranded DNA fragments provided in step (S200). The double-stranded DNA fragment may have a length of between about 50 and 10,000 base pairs. Typically, the DNA fragment is between about 50 and 1,000 base pairs.

The method may first identify steps to prepare the DNA fragments provided in the step (S200) for sequencing. To prepare the DNA fragments for sequencing, the first strand and the second strand of DNA fragments on one of the two ends of DNA fragments are ligated to the first hairpin-oligonucleotide and to the other of the two ends of DNA fragments with the second hairpin oligonucleotide (step (S300A)). A hairpin oligonucleotide is a single-stranded (and therefore one-piece) oligonucleotide with a hairpin structure. A hairpin structure is a stem-loop structure with double-stranded stem and short single-stranded loop. For this purpose, the hairpin oligonucleotide has two mutually complementary sections which form the double-stranded stem and which include an unpaired intermediate section, the unpaired intermediate section forming the single-stranded loop. The loop provides the single-stranded portion of the connected product resulting from step (S300A). The length of the loop is at least about 6 nucleotides. The length of the stem is at least about 6 base pairs. The hairpin oligonucleotide has a total length of about 12 to about 200 nucleotides. The hairpin oligonucleotide may also be called a hairpin adapter.

By connecting the 5' end of first strand with the 3' end of the first hairpin-oligonucleotide and connecting the 3' end of second strand with the 5' end of the second hairpin oligonucleotide, and connecting the 3' end of first strand with the 5' end of the second hairpin oligonucleotide in step (S300A), the first strand and the second strand of DNA fragments become physically combined at the two ends of the DNA fragment. The resulting ligated product is a circular DNA molecule that acts as a circular DNA matrix for Rolling Circle Amplification (RCA) in step (S500A). In this way, a so-called "Rolling Circle Library" can be used for sequencing. The link may be made by known molecular biological ligations.

The first hairpin-oligonucleotide and the second hairpin-oligonucleotide may be different or identical to each other.

In step (S500A), the ligated product obtained in step (S300A) may be amplified by RCA. This step also serves to prepare the DNA fragments provided in step (S200) for sequencing. RCA is a well-known isothermal DNA amplification technique, where DNA polymerase continuously adds individual nucleotides to a primer bound to a circular DNA matrix. This results in a long concatemeric single-stranded DNA molecule that has multiple copies, each of which is complementary to the circular DNA matrix or template.

For performing RCA in step (S500A), the ligated product obtained in step (S300A), which serves as a circular DNA matrix or template, is reacted with DNA polymerase in an appropriate buffer compatible with DNA polymerase and with a primer and nucleotides (deoxynucleotide triphosphates (dNTPs)). The primer is elected such that the primer may bind to the ligated product. Typically, the single-stranded section of the first and second hairpin oligonucleotides each provides a primer binding site for the step (S500A) and the primer is so selected such that the primer may bind to the primer binding site provided by the single section of the first hairpin oligonucleotide and the single section of the second hairpin oligonucleotide. If the first and second hairpin oligonucleotide provide different primer binding sites, two different primers are chosen for RCA, each of which can bind to one of the primer binding sites. Alternatively, only the first hairpin oligonucleotide, or only the second hairpin oligonucleotide, may provide a primer binding site for step (S500A). RCA may be carried out at a constant temperature in the range of about 20°C to about 65°C.

The RCA performed in step (S500A) results in multiple copies that are complementary to the first strand sequence and multiple copies that are complementary to the second strand sequence being arranged on a single-stranded DNA molecule. In so doing, the copies which are complementary to the first strand sequence alternate with the copies that are complementary to the second strand sequence on the single-stranded DNA molecule. This may be referred to as tandem repeats. A sequence that is complementary to the sequence of the first hairpin oligonucleotide or to the sequence of the second hairpin oligonucleotide is arranged between the individual copies.

In step (S600A) the ligated product obtained in step (S300A) or the amplicon received in step (S500A) is amplified. This step also serves to prepare the DNA fragments provided in step (S200) for sequencing. In amplification carried out in step (S600A), redox modified nucleotides, which have pre-specified redox species, may be incorporated into the amplicons. A first group of redox modified nucleotides and a second group of redox modified nucleotides is used for this purpose. Each redox modified nucleotide in the first group has a first redox species with an initial oxidation or reduction potential. The first group consists of redox modified nucleotides of the type of deoxyadenosine triphosphate (dATP) or redox modified nucleotide of the species deoxythymidine triphosphate (dTTP) or redox modified nucleotide of the species deoxyuridine triphosphate (dUTP). Each redox modified nucleotide in the second group has a second redox species with a second oxidation or reduction potential. The second group consists of redox modified nucleotide of the kind of deoxycytidine triphosphate (dCTP) or from redox modified nucleotide of the species deoxyguanosine triphosphate (dGTP). Thus, two types of redox modified nucleotides may be used. Oxidizing or reducing the redox species produces an electrochemical signal specific to a particular type of redox species, so that the electrochemical signal can be used to identify the redox species of the redox-modified nucleotide. The difference in the electrochemical signal of the first redox species and the electrochemical signal of the second redox species *(i.e.* the difference between the first oxidation or reduction potential and the second oxidation or reduction potential) should be sufficiently large to distinguish between the two redox species. The redox species should also be stable under standard laboratory conditions.

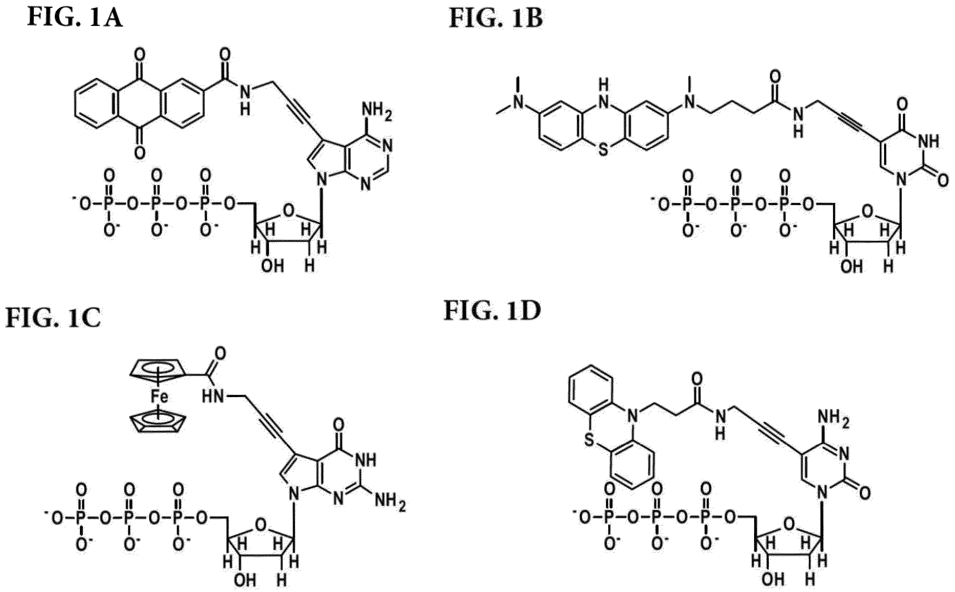

Appropriate redox species and corresponding redox modified nucleotides are known. Examples of suitable redox species are anthraquinone (anthracene-9,10-dione) with an oxidation potential of about 0.40 V, methylene blue (3,7-bis(dimethylamino)-phenothiazin-5-ium chloride) with an oxidation potential of about 0.20 V, ferrocene (cyclopenta -1,3-diene; iron (2 +)) with an oxidation potential of 0.50 V, and phenothiazine (10H phenothiazine) with an oxidation potential of 0.60 V (all potentials against Ag/AgCl). Other examples of suitable redox species may include ferrocene derivatives, osmium and ruthenium complexes, tetrathiafulvalene, aminophenol, nitrophenol, erythrosine B, and the methylene blue derivative ATTO MB2. Examples of redox modified nucleotides are known, inter alia, from WO 2019/086305 A1 and shown in Figs. 1A-1D and Figs. 2A and 2B. Methods of synthesis of redox modified nucleotide are known, i.e., from US 11,814,675 B2.

The nucleotide dATP, dCTP, dGTP, dTTP, and/or dUTP may be the native forms of nucleotides or suitable derivatives thereof, i.e. modified variants thereof.

In a first embodiment of the method, in step (S600A), the ligated product received in step (S300A) may be amplified by RCA. The steps S500A and (S600A) may be performed together. The ligated product obtained in step (S300A) may be amplified by RCA using the first group of redox-modified nucleotides and the second group of redox-modified nucleotides. The nucleotides complementary to the first group of redox-modified nucleotides and the nucleotides complementary to the second group of redox-modified nucleotides may be each used in a non-redox-modified form. Suitable DNA polymerases with strand displacement activity are commercially available. These include, for example, the SD polymerase from Bioron GmbH (Bioron GmbH, Römerberg, Germany), the Bst 3.0 DNA polymerase from New England Biolabs, Inc. (New England Biolabs, Inc., Ipswich, MA, USA), and the Vent (exo-) DNA polymerase from New England Biolabs, Inc. (New England Biolabs, Inc., Ipswich, MA, USA).

A first embodiment of the first method for sequencing of a DNA strand may thus include:
(S200) providing the double-stranded DNA fragment with the first strand having a 5' end and a 3' end and the second strand having a 5' end and a 3' end, wherein the first strand and the second strand are complementary to each other, the DNA strand to be sequenced corresponding to the first strand or the second strand;
(S300A) connecting the 5' end of the first strand to the 3' end of the first hairpin oligonucleotide and connecting the 3' end of the second strand to the 5' end of the first hairpin oligonucleotide, and
connecting the 3' end of the first strand to the 5' end of the second hairpin oligonucleotide and connecting the 5' end of the second strand to the 3' end of the second hairpin oligonucleotide, wherein the first hairpin oligonucleotide and the second hairpin oligonucleotide each provide a single-stranded portion of the resulting ligated product;
(S500A and S600A) amplifying the ligated product obtained in step (S300A) by RCA using the first group of redox-modified nucleotides and the second group of redox-modified nucleotides, wherein each redox-modified nucleotide in the first group has a first redox species with a first oxidation or reduction potential and each redox-modified nucleotide in the second group has a second redox species with a second oxidation or reduction potential. The first group may include, comprise, or consist of redox-modified nucleotides of the type deoxyadenosine triphosphate (dATP), deoxythymidine triphosphate (dTTP), or deoxyuridine triphosphate (dUTP), and the second group includes, comprises, or consists of redox-modified nucleotides of the type deoxycytidine triphosphate (dCTP) or deoxyguanosine triphosphate (dGTP); and
(S800A) determining sequence of DNA strand by sequencing the amplicons obtained in step (S600A) by an electrochemical sequencing method.

In an alternative, second, embodiment of the first method, in step (S600A), the amplicon received in step (S500A) is amplified by primer extension. The step (S600A) may be performed separately after the step (S500A), and the amplification of the amplicon received in step (S500A) may be done using primer extension. In contrast to the first embodiment, the amplification of the related product received in step (S300A) using RCA is performed by RCA in step (S500A) using only non-redox modified nucleotide. For RCA, DNA polymerases with strandulation activity are commercially available. These include, for example, the SD polymerase of Bioron GmbH (Bioron GmbH, Römerberg, Germany), Bst 3.0 DNA polymerase of New England Biolabs, Inc. (New England Biolabs, Inc., Ipswich, MA, USA), and the Vent (exo-) DNA polymerase of New England Biolabs, Inc. (New England Biolabs, Inc, Ipswich, MA, USA).

In step (S600A), the amplicon received in step (S500A) is amplified by primer extension. Primer extension is a known DNA amplification technique in which a DNA polymerase continuously adds individual nucleotides to a primer bound to a DNA template. The amplicons obtained in step (S500A) serve as DNA templates for the primer extension. These are brought together to perform primer extension with a DNA polymerase in a suitable buffer compatible with the DNA polymerase, as well as a primer and dNTPs. Primer extension takes place using the first group of redox-modified nucleotides and the second group of redox-modified nucleotides. The nucleotides complementary to the first group of redox-modified nucleotides and the nucleotides complementary to the second group of redox-modified nucleotides are each used in a non-redox-modified form. Suitable DNA polymerases are commercially available. These include, for example, the SD polymerase from Bioron GmbH (Bioron GmbH, Römerberg, Germany), the Bst 3.0 DNA polymerase from New England Biolabs, Inc. (New England Biolabs, Inc., Ipswich, MA, USA), the Vent (exo-)DNA polymerase from New England Biolabs, Inc. (New England Biolabs, Inc., Ipswich, MA, USA), the Therminator DNA polymerase from New England Biolabs, Inc. (New England Biolabs, Inc., Ipswich , MA, USA), the Novagen KodXL polymerase from Merck KGaA (Merck KGaA, Darmstadt, Germany), the KOD DNA polymerase from Merck KGaA (Merck KGaA, Darmstadt, Germany), the Pwo DNA polymerase from Merck KGaA (Merck KGaA , Darmstadt, Germany) and the Klenow fragment.

The primer is chosen such that it can bind to the amplicons. Typically, the primer is chosen such that it can bind to at least a portion that is complementary to a portion of the sequence of the first and/or second hairpin oligonucleotide.

The second embodiment of the first method for sequencing a DNA strand may therefore include the following steps:
(S200) providing the double-stranded DNA fragment with the first strand having a 5' end and a 3' end and the second strand having a 5' end and a 3' end, wherein the first strand and the second strand are complementary to each other, the DNA strand to be sequenced corresponding to the first strand or the second strand;
(S300A) connecting the 5' end of the first strand to the 3' end of the first hairpin oligonucleotide and connecting the 3' end of the second strand to the 5' end of the first hairpin oligonucleotide, and connecting the 3' end of the first strand to the 5' end of the second hairpin oligonucleotide and connecting the 5' end of the second strand to the 3' end of the second hairpin oligonucleotide, wherein the first hairpin oligonucleotide and the second hairpin oligonucleotide each provide a single-stranded portion of the resulting ligated product;
(S500A) amplifying the related product obtained in step (S300A) by Rolling Circle amplification (RCA);
(S600A) amplifying the amplicons obtained in step (S500A) using primer extension using the first group of redox modified nucleotides and the second group of redox modified nucleotides, each redox modified nucleotide in the first group having a first redox species with a first oxidation or reduction potential and each redox modified nucleotide in the second group having a second oxidation or reduction potential, with the first group of redox modified nucleotide of the type deoxyadenosine triphosphate (dTTP), desoxythymidine triphosphate (dTTP), desoxythymidine triphosphate (dTTP), deoxyguanosine triphosphate (dGTP), or deoxyguanosine triphosphate (dGTP); and
(S800A) determining the sequence of DNA strand by sequencing the amplicons obtained in step (S600A) by an electrochemical sequencing method.

In both embodiments of the first method, the amplification carried out in step (S600A) leads to redox-labeled amplicons, the sequence of which can be determined in the step (S800A) using an electrochemical sequencing method. The redox-labeled amplicons are single-stranded DNA molecules on which there are alternating copies that are complementary to the sequence of the first strand and copies that are complementary to the sequence of the second strand. When using two types of redox-modified nucleotides, the redox-modified nucleotides must be present in both the copies complementary to the first strand sequence and the copies complementary to the second strand sequence identifiable in step (S800A). Due to the complementarity of the first and second strands, the complete sequence of the first strand and the second strand can then be determined by combining the obtained information. The sequence of the initial DNA strand can thus be determined from the sequence of the redox-labeled amplicons.

An electrochemical sequencing method may be used for the sequencing of redox reactions. In the electrochemical sequencing method, the redox-modified nucleotides of the amplicons are oxidized or reduced sequentially along the amplicon. Oxidizing or reducing produces an electrochemical signal that is specific to a particular type of redox species, so the electrochemical signal is used to identify the redox species of the redox-modified nucleotide. Each redox species is specific for a certain type of nucleotide, so that the redox species can be used to determine which type of nucleotide is incorporated at a certain position in the redox-labeled amplicon. The sequencing of the redox-labeled amplicons is based on this principle. If redox-modified nucleotides of the type dUTP are used in step (S600A), the identification of a dUTP is based on the base thymine in the sequence of the redox-labeled amplicon. A gap in the electrochemical signal corresponds to one of the two types of nucleotides used in the step (S600A) in a non-redox modified form. An example of an electrochemical sequencing method is sequencing by electronic tunneling, which is known from WO 2019/086305 A1.

The electrochemical sequencing method known from WO 2019/086305 A1 can, for example, include the following steps: (a) providing a DNA molecule generated using the first group of redox-modified nucleotides and the second group of redox-modified nucleotides; (b) applying a voltage to at least one edge electrode; (c) guiding the DNA molecule over the at least one edge electrode; (d) oxidizing or reducing each redox-modified nucleotide using the at least one edge electrode while the respective redox-modified nucleotide is passed over the at least one edge electrode, wherein the oxidizing or reducing produces an electrochemical signal comprising a change in current; (e) identifying each redox-modified nucleotide based on the electrochemical signal; and (f) determining a sequence of the DNA molecule. The amplicons obtained in step (S600A) of the disclosed method are each capable of being the DNA molecule to be provided in the step (a) of the sequencing method.

The Rolling Circle method allows a complete reconstruction of the sequence of the initial DNA strand with simultaneous determination of the sequence of the DNA strand on the basis of the redox DNA molecules, which have the sequence of first strand and the sequence of the second strand in a complementary form in multiple copies each. This increases the precision of the sequence determination. In addition, the method requires less computing power for the reconstruction of a complete sequence from a single molecule compared to the use of two or more molecules such as those required under the UMI method, as is described further.

In the first embodiment of the first method, the additional step of the amplifying the amplicons obtained using RCA is omitted.

In the second embodiment of the first method, more amplicons are available for step (S800A) compared to the first embodiment.

According to a further embodiment, a third group of redox-modified nucleotide may be used in step (S600A). Each redox-modified nucleotide in the third group has a third redox species with a third oxidation or reduction potential. According to a further embodiment, a fourth class of redox-modified nucleotide may be used in the step (S600A). Each redox-modified nucleotide in the fourth group has a fourth redox species with a fourth oxidation or reduction potential. Thus, three or four types of redox modified nucleotide may be used. Each redox species is specific for a particular type of nucleotide so that the redox species can be used to determine type of nucleotide is incorporated at a certain position in the redox-labeled amplicon. Each group of redox-modified nucleotides therefore has a different type of nucleotide. The difference in the electrochemical signal of the different redox species should be large enough to allow distinction between the redox species.

When using three types of redox-modified nucleotide, each gap in the electrochemical signal corresponds to the type of nucleotide that was used in step (S600A) in a non-redox-modified form. When using four types of redox-modified nucleotides, each nucleotide is redox-modified so that each nucleotide produces an electrochemical signal. Therefore, when using three or four types of redox-modified nucleotides, it is sufficient to identify the redox-modified nucleotides either in the copies complementary to the first strand sequence or in the copies complementary to the second strand sequence in step (S800A).

According to a further embodiment, the method further comprises:
(S400A) selecting a correctly ligated product obtained in step (S300A) using an exonuclease treatment.

An exonuclease breaks down a DNA molecule starting from a free 5' end or a free 3' end. Exonuclease treatment therefore degrades DNA molecules with a free 5' end or a free 3' end. A product correctly connected in step (S300A) has neither a free 5' end nor a free 3' end and is therefore protected from degradation by exonuclease treatment. On the other hand, an incorrectly connected (by-)product is degraded by the exonuclease treatment. In this way, a selection of a product correctly connected in step (S300A) is achieved. An example of an incorrectly ligated byproduct is a DNA fragment that is ligated to a hairpin oligonucleotide at only one of its two ends, while the other of its two ends remains unchanged.

In accordance with another embodiment, the method further comprises:
(S700A) selecting the applications obtained in step (S600A) which include the redox-modified nucleotides.

For selecting the amplicons obtained in step (S600A) which have the redox-modified nucleotides, biotinylated primers may be used, for example, in step (S600A). The biotinylated primers mean that the amplicons containing the redox-modified nucleotides have a biotin molecule and can therefore be selected using streptavidin purification (positive selection). Streptavidin purification can be carried out, for example, using streptavidin-coupled beads. The interaction between streptavidin and biotin allows the selection of the amplicons obtained in step (S600A) that have the redox-modified nucleotides.

The step (S700A) removes DNA molecules that are present at the end of the step (S600A) together with the redox-labeled amplicons, but which do not have redox-labeled nucleotides and thus do not generate electrochemical signals, in step (S800A). These include, for example, the DNA molecules used as DNA matrices in step (S600A). Thus, through step (S700A), the subsequent step (S800A) can be carried out more efficiently.

According to a further embodiment, the method may further include:
(S250) producing overhang sequences in the double-stranded DNA fragment by dA-tailing of first strand and dA-tailing of the second strand.

The term "tailing" refers to the matrix-independent attachment of at least one dNTP to a 3' end of a DNA strand. For this purpose, a terminal transferase is used. Suitable terminal transferases are commercially available. These include, for example, the Klenow fragment (3'→ 5' exo-), the Taq DNA polymerase and the terminal transferase from New England Biolabs, Inc. (New England Biolabs, Inc., Ipswich, MA, USA). In the dA-tailing of the first strand of the double-stranded DNA fragment, at least one dATP is attached to the 3' end of the first strand, and in the dA-tailing of the second strand of the double-stranded DNA fragment, at least one dATP is attached to the 3' end of the second strand. Due to the dA-tailing of the first strand and the dA-tailing of the second strand in step (S250), the DNA fragment has a single-stranded overhang sequence at both ends. As an alternative to step (S250), the double-stranded DNA fragment can already be provided in a form in which the 3' end of the first strand and the 3' end of the second strand each have a single-stranded overhang sequence, with the overhang sequence each consisting of one or more deoxyadenosines.

According to a further embodiment, the method further comprises:
(S260A) creating overhang sequences in the first hairpin oligonucleotide and in the second hairpin oligonucleotide by dT tailing.

When dT tailing the first and second hairpin oligonucleotides, at least one dTTP is attached to the 3' end of the first and the 3' end of the second hairpin oligonucleotide. Due to dT-tailing the first and second hairpin oligonucleotides in step (S260A), the first and second hairpin oligonucleotides each have a single-stranded overhang sequence. As an alternative to step (S260A), the first and second hairpin oligonucleotides may already be provided in a form in which the 3' end of the first hairpin oligonucleotide and the 3' end of the second hairpin oligonucleotide each have a single-stranded overhang sequence, wherein the overhang sequence consists of one or more deoxythymidines.

Base pairing occurs between the overhang sequences of the DNA fragment and the overhang sequences of the first and second hairpin oligonucleotides. Joining of the 5' end of the first strand to the 3' end of the first hairpin oligonucleotide and joining of the 3' end of the second strand to the 5' end of the first hairpin oligonucleotide, as well as joining of the 3' end of the first strand to the 5' end of the second hairpin oligonucleotide and joining of the 3' end of the second hairpin oligonucleotide and joining of the 3' end of the first hairpin oligonucleotide 5' end of the second strand is facilitated with the 3' end of the second hairpin oligonucleotide in step (S300A).Suitable ligases are commercially available. These include, for example, the T4 DNA ligase, the HiFi Taq DNA ligase, and the Thermostable 5' App DNA/RNA ligase from New England Biolabs, Inc. (New England Biolabs, Inc., Ipswich, MA, USA).

Alternatively, joining the 5' end of the first strand to the 3' end of the first hairpin oligonucleotide and joining of the 3' end of the second strand to the 5' end of the first hairpin oligonucleotide, and joining the 3' end of the first strand to the 5' end of the second hairpin oligonucleotide and joining the 5' end of the second strand to the 3' end of the second hairpin oligonucleotide in step (S300A) may be done by blunt end ligation. In this case, no overhang sequences are required. Suitable ligases are commercially available. These include, for example, the T4 DNA ligase, the HiFi Taq DNA ligase, and the thermostable 5' App DNA/RNA ligase of the New England Biolabs, Inc (New England Biolabs, Inc., Ipswich, MA, USA). To avoid undesired by-products in blunt ligation, the first and second hairpin oligonucleotide may be used in excess relative to the DNA fragment.

According to a further embodiment, the method further comprises:
(S100) fragmenting a double-stranded DNA into double-stranded DNA fragments with a first strand having a 5' end and a 3' end and a second strand having a 5' end and a 3' end; and
(S150) performing DNA final repair of the DNA fragments obtained in step (S100).

Methods for fragmenting a double-stranded DNA into double-stranded DNA fragments are known. These include, for example, fragmenting by mechanical shearing (for example with the aid of a thin needle), enzymatic fragmentation (for example with the aid of a transposase, restriction enzymes or nicking enzymes), and the physical fragmentation (for example with the aid of an ultrasound treatment). The DNA fragments produced during fragmentation typically have sticky ends, *i.e.* one strand is longer than the other. DNA fragments with sticky ends need to be converted into DNA fragments with blunt ends. This involves repairing the sticky ends and creating blunt ends. This process is referred to as DNA final repair. By fragmenting and performing DNA final repair, double-stranded DNA fragments can be generated which can be provided in step (S200). By determining the sequence of several of the DNA fragments, it is possible to determine the sequence of the double-stranded DNA, or at least one longer portion of the double-stranded DNA.

The second method for sequencing of a DNA strand comprises:
(S200) providing a double-stranded DNA fragment having a first strand having a 5' end and a 3' end and a second strand having a 5' end and a 3' end, wherein the first strand and the second strand are complementary to one another, wherein the DNA strand to be sequenced corresponds to the first strand or the second strand;
(S300B) connecting the 5' end of the first strand and the 3' end of the second strand to a first UMI oligonucleotide and connecting the 3' end of the first strand and the 5' end of the second strand to a second UMI oligonucleotide, wherein the first UMI oligonucleotide and the second UMI oligonucleotide are each two-stranded, Y-shaped oligonucleotides having a paired end portion and an unpaired end portion. The paired end portion is connected to the first strand and the second strand. The paired end portion is connected to the UMI sequence of the first UMI oligonucleotide and the second strand is connected to the UMI sequence of the second UMI oligonucleotide by connecting the first strand to the UMI sequence of the first UMI oligonucleotide;
(S500B) amplifying the ligated product obtained in step (S300B) by polymerase chain reaction (PCR);
(S600B) amplifying the amplicons obtained in step (S500B) by primer extension using a first group of redox-modified nucleotides and a second group of redox-modified nucleotides, wherein each redox-modified nucleotide in the first group comprises a first redox species having a first oxidation or reduction potential, and each redox-modified nucleotide in the second group comprises a second redox species having a second oxidation or reduction potential, wherein the first group consists of redox-modified nucleotides of the type deoxyadenosine triphosphate (dATP), deoxythymidine triphosphate (dTTP), or deoxyuridine triphosphate (dUTP), and the second group consists of redox-modified nucleotides of the type deoxycytidine triphosphate (dCTP) or deoxyguanosine triphosphate (dGTP); and
(S800B) determining the sequence of the DNA strand by sequencing the amplicons obtained in step (S600B) using an electrochemical sequencing method.

The second method for sequencing of a DNA strand is also referred to below as UMI method.

A DNA strand is sequenced using the method, *i.e.* a nucleotide sequence (sequence) of the DNA strand is determined. The DNA strand is also referred to below as an initial DNA strand. The DNA strand corresponds to the first strand or the second strand of the double-stranded DNA fragment provided in step (S200). The double-stranded DNA fragment may have a length of about 50 to about 10,000 base pairs. Typically, the DNA fragment has between about 50 and about 1,000 base pairs.

The method initially includes steps for preparing the DNA fragment provided in step (S200) for sequencing. To prepare the DNA fragment for sequencing, the DNA fragment is connected at its two ends to, in each case, one UMI oligonucleotide (step S300B). A UMI oligonucleotide is a two-stranded, Y-shaped oligonucleotide. The Y shape is due to the UMI oligonucleotide having a paired end portion and an unpaired end portion. In the paired end portion, the sequences of the two strands of the UMI oligonucleotide are complementary to each other. On one strand, the paired end portion has a unique molecular identification sequence (UMI sequence) which is arranged at the terminal end. By means of the terminal arrangement of the UMI sequence, the UMI sequence can be connected to a strand of the DNA fragment. The term "UMI" stands for a unique molecular identifier. The UMI sequence consists of a predetermined unique random sequence of nucleotides. The UMI sequence can also be referred to as a barcode sequence or as a molecular bar code. The UMI sequence has a length of at least about 10 nucleotides, preferably of at least about 40 nucleotides, up to about 100 nucleotides. The UMI sequence of the first UMI oligonucleotide differs from the UMI sequence of the second UMI oligonucleotide. On the basis of the UMI sequence, the UMI oligonucleotides allow a unique identification of the first strand and of the second strand and thus an unambiguous assignment of the first strand to the second strand after sequencing. The unique assignment of the first strand to the second strand after sequencing is particularly important when only two types of redox-modified nucleotides are used. In this case, the information about the redox-modified nucleotides identified in the first strand and via the redox-modified nucleotides identified in the second strand must be combined to determine the complete sequence of the first strand and the second strand.

The paired end portion may include a further double-stranded portion adjacent to the portion having the UMI sequence and the complementary sequence thereof. This further double-stranded portion has a length of about 10 to about 40 base pairs and is disposed between the portion having the UMI sequence and the complementary sequence thereto and the unpaired end portion of the UMI oligonucleotide.

The paired end portion has a length of about 10 to about 140 base pairs.

In the unpaired end portion, the sequences of the two strands of the UMI oligonucleotide are not complementary to one another. The unpaired end portion may also be referred to as an asymmetric flanking site. The unpaired end portion has a length of from about 10 to about 40 nucleotides per strand. The unpaired end portion of the first UMI oligonucleotide and the unpaired end portion of the second UMI oligonucleotide may be identical to each other.

The UMI oligonucleotide generally has a length of from about 20 to about 180 nucleotides per strand. The UMI oligonucleotide can also be referred to as UMI adapter.

In step (S300B), the first strand and the second strand of the DNA fragment remain only non-physically connected to one another, but the first strand and the second strand can be uniquely identified and assigned to one another by the UMI oligonucleotides after sequencing.

Connecting the 5' end of the first strand and the 3' end of the second strand to the paired end portion of the first UMI oligonucleotide and connecting the 3' end of the first strand and the 5' end of the second strand to the paired end portion of the second UMI oligonucleotide in step (S300B) leads to a ligated product which functions as a DNA template for the polymerase chain reaction (PCR) in step (S500B). In this way, a so-called "UMI library" may be prepared for sequencing. The connection may be carried out using known molecular biological ligation processes.

In step (S500B), the product obtained in step (S300B) is amplified by PCR. This step also serves to prepare the DNA fragment provided in step (S200) for sequencing. The PCR is a known DNA amplification technique leading to a plurality of copies of a DNA portion of a DNA template. The PCR has several cycles, each cycle consisting of the three steps of denaturation, primer binding, and primer elongation. The three steps take place at different temperatures. During primer elongation, a DNA polymerase continuously attaches individual nucleotides to the primer.

To perform PCR in step (S500B), the ligated product obtained in step (S300B), which serves as a DNA template, is combined with a DNA polymerase in a suitable buffer compatible with the DNA polymerase and with a primer and dNTPs. The primer is chosen such that it can bind to the ligated product. Typically, the unpaired end portion of the first and second UMI oligonucleotide each provides a primer binding site for step (S500B). The primer is selected to bind to the primer binding site provided by the unpaired end portion of the first UMI oligonucleotide and the unpaired end portion of the second UMI oligonucleotide. Alternatively, a first portion of the primer binding site on the unpaired end portion and a second portion of the primer binding site may be located on the paired end portion of the first and/or second UMI oligonucleotide. If the first and the second UMI oligonucleotide provide different primer binding sites, two different primers are selected for PCR, each of which can bind to one of the primer binding sites.

The PCR carried out in step (S500B) results in multiple amplicons of the ligated product, wherein the primer binding site respectively marks the beginning or end of each amplicon, and the amplicons are double-stranded over their entire length. The multiple amplicons can also be referred to as a so-called "UMI clone."

In step (S600B), the amplicons obtained in step (S500B) are amplified primer extension. This step also serves to prepare the DNA fragment provided in step (S200) for sequencing. In the amplification carried out in step (S600B), redox-modified nucleotides which have predetermined redox species are incorporated into the amplicons. For this purpose, a first group of redox-modified nucleotides and a second group of redox-modified nucleotides are used. Each redox-modified nucleotide in the first group has a first redox species having a first oxidation or reduction potential. The first group consists of redox-modified nucleotides of the type deoxyadenosine triphosphate (dATP), deoxythymidine triphosphate (dTTP), or deoxyuridine triphosphate (dUTP). Each redox-modified nucleotide in the second group has a second redox species with a second oxidation or reduction potential. The second group consists of redox-modified nucleotides of the type deoxycytidine triphosphate (dCTP) or deoxyguanosine triphosphate (dGTP). Thus, two types of redox-modified nucleotides are used. Oxidizing or reducing the redox species produces an electrochemical signal that is specific to a particular type of redox species so that the redox species of the redox-modified nucleotide can be identified based on the electrochemical signal. The difference in the electrochemical signal of the first redox species and the electrochemical signal of the second redox species (*i.e.,* the difference between the first oxidation or reduction potential and the second oxidation or reduction potential) should be sufficiently large to allow distinction between the two redox species. The redox species should also be stable under standard laboratory conditions. Suitable redox species and corresponding redox-modified nucleotides are known. The examples given in the description of the Rolling Circle Method for this also apply to the UMI method.

The nucleotides dATP, dCTP, dGTP, dTTP, and/or dUTP can each be the native forms of the nucleotides or suitable derivatives thereof, *i.e.* modified variants thereof.

In step (S600B), the amplicons obtained in step (S500B) are amplified primer extension. The primer extension is a known DNA amplification technique in which a DNA polymerase continuously attaches individual nucleotides to a primer bound to a DNA template. The amplicons obtained in step (S500B) serve as DNA matrices for the primer extension. These are brought together for carrying out the primer extension with a DNA polymerase in a suitable buffer compatible with the DNA polymerase and with a primer and dNTPs. The primer extension takes place using the first group of redox-modified nucleotides and the second group of redox-modified nucleotides. The nucleotides complementary to the first group of redox-modified nucleotides and the nucleotides complementary to the second group of redox-modified nucleotides are each used in a non-redox-modified form. Suitable DNA polymerases are commercially available. These include, for example, the SD polymerase from Bioron GmbH (Bioron GmbH, Römerberg, Germany), the Bst 3.0 DNA polymerase from New England Biolabs, Inc. (New England Biolabs, Inc., Ipswich, MA, USA), the Vent (exo-) DNA polymerase from New England Biolabs, Inc. (New England Biolabs, Inc., Ipswich, MA, USA), the Therminator DNA polymerase from New England Biolabs, Inc. (New England Biolabs, Inc., Ipswich, MA, USA), the Novagen KodXL polymerase from Merck KGaA (Merck KGaA, Darmstadt, Germany), the KOD DNA polymerase from Merck KGaA (Merck KGaA, Darmstadt, Germany), the Pwo DNA polymerase from Merck KGaA (Merck KGaA, Darmstadt, Germany), and the Klenow fragment.

The primer is chosen such that it can bind to the amplicons obtained in step (S500B). Typically, the primer is chosen such that it can bind to the primer binding sites that were already used for PCR in step (S500B) and which are therefore located at the beginning or at the end of the amplicons.

The amplification performed in step (S600B) results in redox-labeled amplicons, the sequence of which can be determined in step (S800B) using an electrochemical sequencing method. The redox-labeled amplicons are single-stranded or double-stranded DNA molecules, which can be considered redo-labeled copies of the UMI clone. Each redox-labeled single strand contains either one copy of the first strand of the DNA fragment or one copy of the second strand of the DNA fragment. The redox-labeled single strands, which comprise one copy of the first strand of the DNA fragment, have the UMI sequence of the first UMI oligonucleotide, as well as the sequence complementary to the UMI sequence of the second UMI oligonucleotide. The redox-labeled single strands, which comprise one copy of the second strand of the DNA fragment, have the UMI sequence of the second UMI oligonucleotide, as well as the sequence complementary to the UMI sequence of the first UMI oligonucleotide. As a result, the first strand and the second strand of the DNA fragment can be clearly assigned to each other after sequencing. When using two types of redox-modified nucleotides, the redox-modified nucleotides must be identified in both the copies of the first strand and the copies of the second strand in step (S800B). Due to the complementarity of the first and second strands, the complete sequence of the first strand and the second strand can then be determined by merging the information obtained. Thus, the sequence of the initial DNA strand can be determined from the sequence of the redox-labeled amplicons. In addition, by using the UMI oligonucleotides, the sequences of the copies of the first strand and the sequences of the copies of the second strand can be clearly assigned to each other and matched with each other after sequencing to determine the consensus sequence of the DNA strand.

An electrochemical sequencing method may be used for the sequencing of redox-labeled amplicons. The further example in the description of the Rolling Circle method also apply to the UMI method.

The UMI method allows a complete reconstruction of the sequence of the initial DNA strand. The method also makes it possible to determine the consensus sequence of the DNA strand by comparing the results of several readouts showing an identical UMI sequence. This increases the accuracy of sequence determination.

According to a further embodiment, a third group of redox-modified nucleotides may be used in step (S600B). Each redox-modified nucleotide in the third group has a third redox species with a third oxidation or reduction potential. According to another embodiment, a fourth class of redox-modified nucleotides may be used in step (S600B). Each redox-modified nucleotide in the fourth group has a fourth redox species with a fourth oxidation or reduction potential. Thus, three or four types of redox-modified nucleotides may be used. Each redox species is specific to a specific type of nucleotide so that the redox species can be traced back to what type of nucleotide is incorporated at a certain position in the redox-labeled amplicons. Therefore, each group of redox-modified nucleotides has a different type of nucleotide. The difference in the electrochemical signal from the different redox species should be large enough to allow distinction between the redox species.

When using three types of redox-modified nucleotides, each gap in the electrochemical signal corresponds to the type of nucleotide used in step (S600B) in a non-redox-modified form. Using four types of redox-modified nucleotides, each nucleotide is redox-modified, so each nucleotide produces an electrochemical signal. Therefore, when using three or four types of redox-modified nucleotides, it is sufficient to identify the redox-modified nucleotides either in the copies of the first strand or in the copies of the second strand in the step (S800B).

According to another embodiment, the method further includes:
(S700B) selecting the amplicon obtained in step (S600B) that have the redox-modified nucleotides.

For example, biotinylated primers can be used in step (S600B) to select the amplicons obtained in step (S600B) that have the redox-modified nucleotides. The biotinylated primers lead to the fact that the amplicons of the redox-modified nucleotides have a biotin molecule and can therefore be selected using streptavidine purification (positive selection). For example, streptavidin purification can be done using streptavidin-coupled beads. The interaction between streptavidin and biotin allows the selection of the amplicons obtained in step (S600B) that have the redox-modified nucleotides.

The step (S700B) removes DNA molecules that are present together with the redox-labeled amplicons at the end of step (S600B), but which do not have redox-labeled nucleotides and thus do not generate electrochemical signals, in step (S800B). Thus, through step (S700B), the subsequent step (S800B) can be carried out more efficiently.

A selective double-stranded amplicon can be sequenced directly, as only one strand of amplification is redox-labeled. Alternatively, the unlabeled strand of the double-stranded amplicon may be denatured and removed from the redox-labeled strand exhibiting the streptavidin-biotin interaction so that only the redox-labeled single-strand is sequenced.

According to a further embodiment, the method further includes:
(S250) producing overhang sequences in the double-stranded DNA fragment by dA-tailing of the first strand and dA-tailing of the second strand.

The example disclosed in the description of the Rolling Circle method also apply to the UMI method.

According to a further embodiment, the method further includes:
(S260B) preparing overhang sequences in the first UMI oligonucleotide and in the second UMI oligonucleotide by dT-tailing.

The examples disclosed in the description of the Rolling Circle method also apply to the UMI method.

Alternatively, joining the 5' end of the first strand and the 3' end of the second strand to the first UMI oligonucleotide and joining the 3' end of the first strand and the 5' end of the second strand to the second UMI oligonucleotide in step (S300B) can be done by blunt ligation. In this case, no overhang sequences are required. Suitable ligases are commercially available. The examples disclosed in the description of the Rolling Circle method also apply to the UMI method.

According to a further embodiment, the method further comprises:
(S100) fragmenting a double-stranded DNA into double-stranded DNA fragments with a first strand having a 5' end and a 3' end, and a second strand having a 5' end and a 3' end; and
(S150) performing a final DNA repair of the DNA fragments obtained in step (S100).

The examples disclosed in the description of the Rolling Circle method also apply to the UMI method.

The third method of sequencing a DNA strand includes:
(S200) providing a double-stranded DNA fragment with a first strand having a 5' end and a 3' end, and a second strand having a 5' end and a 3' end, the first strand and the second strand being complementary to each other, wherein the DNA strand to be sequenced corresponds to the first strand or the second strand;
(S300C) connecting the 5' end of the first strand and the 3' end of the second strand to a first oligonucleotide and connecting the 3' end of the first strand and the 5' end of the second strand to a second oligonucleotide, one of the first and second oligonucleotide being a hairpin oligonucleotide and the other of the first and second oligonucleotide being a Y-shaped oligonucleotide, wherein the hairpin oligonucleotide provides a single-stranded portion of the resulting bonded product, wherein the Y-shaped oligonucleotide is a two-stranded oligonucleotide with a paired end portion and an unpaired end portion, wherein the paired end portion is connected to the first strand and the second strand;
(S600C) amplifying the ligated product obtained in step (S300C) by primer extension using a first group of redox-modified nucleotides and a second group of redox-modified nucleotides, wherein each redox-modified nucleotide in the first group has a first redox species with a first oxidation or reduction potential, and each redox-modified nucleotide in the second group has a second redox species with a second oxidation or reduction potential. The first group includes redox-modified nucleotides of the type deoxyadenosine triphosphate (dATP), deoxythymidine triphosphate (dTTP), or deoxyuridine triphosphate (dUTP), and the second group includes redox-modified nucleotides of the type deoxycytidine triphosphate (dCTP), or deoxyguanosine triphosphate (dGTP); and
(S800C) determining sequence of the DNA strand by sequencing the amplicons obtained in step (S600C) using an electrochemical sequencing method.

The third method for the sequencing of a DNA strand may also be called the 2D method.

The method sequences a DNA strand, *i.e.* a nucleotide sequence (sequence) of the DNA strand is determined. The DNA strand is also referred to as the initial DNA strand. The DNA strand corresponds to the first strand or the second strand of the double-stranded DNA fragment provided in step (S200). The double-stranded DNA fragment can have a length of about 50 to about 10,000 base pairs. Typically, the DNA fragment has between about 50 and about 1,000 base pairs.

The method first identifies steps to prepare the DNA fragments provided in the step (S200) for sequencing. To prepare the DNA fragment for sequencing, the DNA fragment is joined at each end with an oligonucleotide (step (S300C). One end of the DNA fragment is connected to a first oligonucleotide and the other end of the DNA fragment to a second oligonucleotide that is structurally different from the first oligonucleotide. One of the first and second oligonucleotides is a hairpin oligonucleotide and the other of the first and second oligonucleotide is a Y-shaped oligonucleotide.

A hairpin oligonucleotide is a single-stranded (and therefore one-piece) oligonucleotide that has a hairpin structure. A hairpin structure is a trunk-loop structure with a double-stranded trunk and a short single-stranded loop. The hairpin oligonucleotide has two complementary sections, which form the double-stranded stem, and which include an unpaired intermediate section forming the single-stranded loop. The loop provides the single-strand portion of the connected product resulting from the step (S300C). The length of the loop is at least 6 nucleotides. The length of the trunk is at least 6 base pairs. The hairpin oligonucleotide has a total length of about 12 to about 200 nucleotides. The hairpin oligonucleotide can also be called a hairpin adapter.

A Y-shaped oligonucleotide is a two-stranded oligonucleotide. The Y-shape is due to the fact that the oligonucleotide has a paired end portion and an unpaired end portion. In the paired final segment, the sequences of the two strands of the Y-shaped oligonucleotide are complementary to each other. The paired end portion has a length of about 10 to about 140 base pairs. In the unpaired end portion, the sequences of the two strands of the Y-shaped oligonucleotide are not complementary to each other. The unpaired end portion can also be called an asymmetrical flanking site. The unpaired end portion has a length of about 10 to about 40 nucleotides per strand. The Y-shaped oligonucleotide has a total length of about 20 to about 180 nucleotides per strand. The Y-shaped oligonucleotide can also be referred to as a Y-shaped adapter.

By connecting the 5' end of the first strand and the 3' end of the second strand to the first oligonucleotide and connecting the 3' end of the first strand and the 5' end of the second strand to the second oligonucleotide in step (S300C), the first strand and the second strand of the DNA fragment are physically connected via the hairpin oligonucleotide at one end. In step (S600C), the resulting ligated product acts as a DNA template for the primer extension. In this way, a so-called "two-dimensional library" ("2D library") can be produced for sequencing. Because the first oligonucleotide and the second oligonucleotide are structurally different, the ligated product can also be called an asymmetrically ligated product. The linking may be carried out by well-known molecular biological ligation methods.

In step (S600C), the ligated product obtained in step (S300C) is amplified. This step is also used to prepare the DNA fragment provided in step (S200) for sequencing. In the amplification performed in step (S600C), redox-modified nucleotides that have predetermined redox species are incorporated into the amplicons. For this purpose, a first group of redox-modified nucleotides and a second group of redox-modified nucleotides are used. Each redox-modified nucleotide in the first group has a first redox species with an initial oxidation or reduction potential. The first group consists of redox-modified nucleotides of the species deoxyadenosine triphosphate (dATP), deoxythymidine triphosphate (dTTP), or deoxyuridine triphosphate (dUTP). Each redox-modified nucleotide in the second group has a second redox species with a second oxidation or reduction potential. The second group consists of redox-modified nucleotides of the species deoxycytidine triphosphate (dCTP) or deoxyguanosine triphosphate (dGTP). So, two types of redox-modified nucleotides are used. Oxidizing or reducing the redox species produces an electrochemical signal that is specific to a particular type of redox species, so the electrochemical signal can be used to identify the redox species of the redox-modified nucleotide. Accordingly, the difference in the electrochemical signal of the first redox species and the electrochemical signal of the second redox species (*i.e.* the difference between the first oxidation or reduction potential and the second oxidation or reduction potential) should be large enough to allow distinction between the two redox species. The redox species should also be stable under standard laboratory conditions. Suitable redox species and corresponding redox-modified nucleotides are known. The examples disclosed in the description of the Rolling Circle method also apply to the 2D method.

The nucleotides dATP, dCTP, dGTP, dTTP, and/or dUTP may be the native forms of nucleotides or suitable derivatives thereof, *i.e.* modified variants thereof.

In the step (S600C), the product ligated in step (S300C) is amplified using primer extension. Primer extension is a well-known DNA amplification technique in which a DNA polymerase continuously attaches individual nucleotides to a primer bound to a DNA template. The DNA template for the primer extension is the ligated product obtained in step (S300C). This is brought together with a DNA polymerase in a suitable buffer that is compatible with the DNA polymerase as well as with a primer and dNTPs to perform primer extension. Primer extension takes place using the first group of redox-modified nucleotides and the second group of redox-modified nucleotides. The nucleotides complementary to the first group of redox-modified nucleotides and the nucleotides complementary to the second group of redox-modified nucleotides are each used in a non-redox-modified form. Suitable DNA polymerases are commercially available. These include, for example, the SD polymerase from Bioron GmbH (Bioron GmbH, Römerberg, Germany), the Bst 3.0 DNA polymerase from New England Biolabs, Inc. (New England Biolabs, Inc., Ipswich, MA, USA), the vent (exo-) DNA polymerase from New England Biolabs, Inc. (New England Biolabs, Inc., Ipswich, MA, USA), the therminator DNA polymerase from New England Biolabs, Inc. (New England Biolabs, Inc., Ipswich, MA, USA), the Novagen KodXL polymerase from Merck KGaA (Merck KGaA, Darmstadt, Germany), the KOD DNA polymerase from Merck KGaA (Merck KGaA, Darmstadt, Germany), the Pwo DNA polymerase from Merck KGaA (Merck KGaA, Darmstadt, Germany), and the Klenow fragment.

The primer is chosen such that it can bind to the product connected in step (S300C). Typically, the unpaired end portion of the Y-shaped oligonucleotide provides a primer binding site for step (S600C) and the primer is selected such that it can bind to the primer binding site provided by the unpaired end portion of the Y-shaped oligonucleotide. Alternatively, a first portion of the primer binding site may be located on the unpaired end portion, and a second portion of the primer binding site may be located on the paired end portion of the Y-shaped oligonucleotide.

The amplification performed in step (S600C) results in multiple redox-labeled amplicons of the ligated product, whereas the sequence of redox-labeled amplicons in step (S800C) can be determined by electrochemical sequencing. The redox-labeled amplicons are single-stranded or double-stranded DNA molecules. Each redox-labeled single strand comprises a single copy that is complementary to the sequence of the first strand and a single copy that is complementary to the sequence of the second strand. When using two types of redox-modified nucleotides, the redox-modified nucleotides must be identified in step (S800C) in both the copies that are complementary to the sequence of the first strand and the copies that are complementary to the sequence of the second strand. Due to the complementarity of the first and second strands, the complete sequence of the first strand and the second strand can then be determined by merging the obtained information. Thus, the sequence of the initial DNA strand can be determined from the sequence of the redox-labeled amplicons.

For the sequencing of the redox-labeled amplicons, an electrochemical sequencing method may be used. The examples disclosed in the description of the Rolling Circle method also apply to the 2D method.

The 2D method enables a complete reconstruction of the sequence of the initial DNA strand. In addition, the method requires less computing power for the reconstruction of a complete sequence from a single molecule compared to the use of two or more molecules such as those required under the UMI method.

According to another embodiment, the paired end portion of the Y-shaped oligonucleotide on a strand has a unique molecular identification sequence (UMI sequence) arranged terminally. Due to the terminal arrangement of the UMI sequence, the UMI sequence can be connected to a strand of the DNA fragment.

According to another embodiment, hairpin oligonucleotide has a unique molecular identification sequence (UMI sequence) that is terminally arranged. Due to the terminal arrangement of the UMI sequence, the UMI sequence can be connected to a strand of the DNA fragment.

It is also possible that both the Y-shaped oligonucleotide and the hairpin oligonucleotide each have a UMI sequence.

The term "UMI" stands for unique molecular identifier. The UMI sequence consists of a predetermined, unique random sequence of nucleotides. The UMI sequence can also be referred to as a barcode sequence or a molecular barcode. The UMI sequence has a length of at least about 10 nucleotides, preferably at least about 40 nucleotides, up to about 100 nucleotides.

The use of the UMI sequence makes it possible to determine the consensus sequence of the DNA strand by comparing the results of multiple readouts showing an identical UMI sequence. This increases the accuracy of sequence determination.

The paired end portion of the Y-shaped oligonucleotide may include another double-stranded segment adjacent to the section that has the UMI sequence and the sequence that complements it. This further double-stranded segment ranges in length from about 10 to about 40 base pairs and is located between the section containing the UMI sequence and the complementary sequence and the unpaired end portion of the UMI oligonucleotide.

According to another embodiment, a third group of redox-modified nucleotides may be also used in step (S600C). Each redox-modified nucleotide in the third group has a third redox species with a third oxidation or reduction potential. According to another embodiment, a fourth group of redox-modified nucleotides may also be used in step (S600C). Each redox-modified nucleotide in the fourth group has a fourth redox species with a fourth oxidation or reduction potential. Therefore, three or four types of redox-modified nucleotides may be used. Each redox species is specific to a specific type of nucleotide, so that the redox species can be used to infer which type of nucleotide is incorporated at a certain position in the redox-labeled amplicon. Each group of redox-modified nucleotides therefore has a different type of nucleotide. The difference in the electrochemical signal of the different redox species should be large enough to allow distinction between the redox species.

When using three types of redox-modified nucleotides, each gap in the electrochemical signal corresponds to the type of nucleotide used in step (S600C) in a non-redox-modified form. Using four types of redox-modified nucleotides, each nucleotide is redox-modified, so each nucleotide produces an electrochemical signal. Therefore, when using three or four types of redox-modified nucleotides, it is sufficient to identify the redox-modified nucleotides either in the copy that is complementary to the sequence of the first strand or in the copy that is complementary to the sequence of the second strand in step (S800C).

According to another embodiment, the procedure also includes:
(S700C) selecting the amplicons obtained in step (S600C) that have the redox-modified nucleotides.

For example, biotinylated primers can be used in step (S600C) to select the amplicons obtained in step (S600C) that have the redox-modified nucleotides. The biotinylated primers lead to the fact that the amplicons of the redox-modified nucleotides have a biotin molecule and can therefore be selected using streptavidine purification (positive selection). For example, streptavidin purification can be done using streptavidin-coupled beads. The interaction between streptavidin and biotin allows the selection of the amplicons obtained in step (S600C) that have the redox-modified nucleotides.

The step (S700C) removes DNA molecules that are present together with the redox-labeled amplicons at the end of step (S600C), but which do not have redox-labeled nucleotides and thus do not generate electrochemical signals, in step (S800C). Thus, through step (S700AC), the subsequent step (S800C) can be carried out more efficiently.

A selected double-stranded amplicon can be sequenced directly, as only one strand of the amplicon is redox-labeled. Alternatively, the unlabeled strand of the double-stranded amplicon can be denatured and removed from the redox-labeled strand exhibiting the streptavidin-biotin interaction so that only the redox-labeled single-strand is sequenced.

According to another embodiment, the procedure also includes:
(S400C) selecting a correctly ligated product, obtained in step (S300C), using a first capture oligonucleotide binding to a specific unique segment of the first oligonucleotide and using a second capture oligonucleotide binding to a specific unique segment of the second oligonucleotide.

For the selection of a correctly ligated product obtained in step (S300C), the first scavenger oligonucleotide binding to a specific unique segment of the first oligonucleotide can be used in a first stage of the selection process in step (S400C). For example, the first catcher oligonucleotide can be a biotinylated first catcher oligonucleotide. Any product associated with the first oligonucleotide can then be selected using streptavidin purification (positive selection). For example, streptavidin purification can be done using streptavidin-coupled beads. The interaction between streptavidin and biotin allows the selection of any product associated with the first oligonucleotide. In a second stage of the selection process in step (S400C), the selected products from the first stage as well as the second scavenger oligonucleotide, which binds to a specific unique section of the second oligonucleotide, are used. For example, the second catcher oligonucleotide can be a biotinylated second catcher oligonucleotide. Each selected product from the first stage that is associated with the second oligonucleotide can then be selected by streptavidine purification (positive selection). For example, streptavidin purification can be done using streptavidin-coupled beads. The interaction between streptavidin and biotin allows the selection of each selected product from the first stage that is associated with the second oligonucleotide. Thus, each selected product from the second stage is associated with both the first oligonucleotide and the second oligonucleotide, *i.e.* each selected product from the second stage is a correctly bonded product. In this way, a product that is correctly connected in the step (S300C) is selected.

An example of an improperly ligated byproduct is a DNA fragment that is only connected to an oligonucleotide at one of its two ends, while the other of its two ends is unchanged. Another example of an improperly bonded byproduct is a DNA fragment that is connected to the same type of oligonucleotide at both ends (*i.e.,* both ends are connected to Y-shaped oligonucleotides or both ends are connected to hairpin oligonucleotides).

According to another embodiment, the procedure also includes:
(S450C) amplifying the ligated product selected in step (S400C) by polymerase chain reaction (PCR).

PCR is a well-known DNA amplification technique that results in a large number of copies of a DNA segment of a DNA template or matrix. To perform PCR in step (S450C), the ligated product selected in step (S400C), which serves as a DNA template or matrix, is brought together with a DNA polymerase in a suitable buffer compatible with the DNA polymerase, as well as with a primer pair and dNTPs. The primer pair is chosen in such a way that the primers can bind to the selected product. Typically, each strand of the unpaired end portion of the Y-shaped oligonucleotide provides a primer binding site for step (S450C), and the primer pair is chosen in such a way that each primer can bind specifically to one of the two primer binding sites. Alternatively, a first section of one or both primer binding sites may be placed on the unpaired end portion, and a second section of one or both primer binding sites may be placed on the paired end portion of the Y-shaped oligonucleotide.

The PCR performed in step (S450C) results in multiple amplicons of the selected product, with the primer binding sites marking the beginning and end of each amplicon respectively, and the amplicons being double-stranded over their entire length. Each individual strand of the amplicons comprises both one copy of the first strand of the DNA fragment and one copy of the second strand of the DNA fragment. In other words, the selected correctly ligated products are enriched by step (S450C).

The amplicons obtained in step (S450C) are amplicons of the product ligated in step (S300C) and can therefore, like the product ligated in step (S300C) itself, be amplified in step (S600C) by primer extension using the first group of redox-modified nucleotides and the second group of redox-modified nucleotides.

According to another embodiment, the procedure also includes:
(S250) producing overhang sequences in the double-stranded DNA fragment by dA-tailing of the first strand and dA-tailing of the second strand.

The examples disclosed in the description of the Rolling Circle method also apply to the 2D method.

According to another embodiment, the procedure also includes:
(S260C) preparing of overhang sequences in the first oligonucleotide and in the second oligonucleotide by dT-tailing.

The examples disclosed in the description of the Rolling Circle method also apply to the 2D method.

Alternatively, the 5' end of the first strand and the 3" end of the second strand are joined to the first oligonucleotide and the 3' end of the first strand and the 5' end of the second strand are joined to the second oligonucleotide in step (S300) by blunt ligation. In this case, no overhang sequences are required. Suitable ligases are commercially available. The examples disclosed in the description of the Rolling Circle method also apply to the 2D method.

According to another embodiment, the procedure also includes:
(S100) fragmenting a double-stranded DNA into double-stranded DNA fragments with a first strand having a 5' end and a 3' end and a second strand having a 5' end and a 3' end, and
(S150) performing a final DNA repair of the DNA fragments obtained in step (S100).

The examples disclosed in the description of the Rolling Circle method also apply to the 2D method.

All of the methods described above for sequencing a DNA strand have the common feature that the sequencing of the redox-labeled amplicons does not take place during the production of the same, but after their production, that the redox-labeled amplicons remain intact during sequencing, and that the sequence of the redox-labeled amplicons is used to determine the sequence of the initial DNA strand.

All of the DNA strand sequencing methods described above can also be used to sequence an RNA strand by transcribing the RNA into complementary DNA (cDNA) by reverse transcription in a step prior to the procedure. Reverse transcription is a well-known molecular biological process.

The DNA strand or RNA strand to be sequenced can come from a biological sample. Methods for isolating DNA or RNA from a biological sample are known.

First reference is made to Figures 1 and 2, which show examples of redox modified nucleotides presented in the methods for sequencing of a DNA strand.

A process sequence of the first method for sequencing of a DNA strand is explained with reference to Figs. 3, 4, and 9A. The first method can also be referred to as the Rolling Circle method.

In a first step (S200), the DNA strand to be sequenced is provided as a strand of a double-stranded DNA fragment 10. The double-stranded DNA fragment 10 consists of a first strand 20 and a second strand 20', where the first strand 20 and the second strand 20' being complementary to each other, and where the DNA strand to be sequenced corresponds to the first strand 20 or the second strand 20. In the present non-limiting example, the DNA strand to be sequenced corresponds to the first strand 20. The first strand 20 has a 5' end and a 3' end. The second strand, 20' also has a 5' end and a 3' end.

In a further step (S250), a dA-tailing of the first strand 20 is performed, in which a dATP is attached to the 3' end of the first strand 20, and a dA-tailing of the second strand 20' is performed, in which a dATP is attached to the 3' end of the second strand 20'. In this way, 10 overhang sequences 21, each consisting of a single deoxyadenosine, are produced at the two ends of the DNA fragment.

In a further step (S260A), a dT-tailing of a first hairpin oligonucleotide 30a is performed, in which a dTTP is attached to a 3' end of the first hairpin oligonucleotide 30a, and a dT-tailing of a second hairpin oligonucleotide 30b is performed, in which a dTTP is attached to a 3' end of the second hairpin oligonucleotide 30b. In this way, overhang sequences 31 are produced at the 3' end of the first hairpin oligonucleotide 30a and the 3' end of the second hairpin oligonucleotide 30b, each consisting of a single deoxythymidine.

Steps (S250) and (S260A) may be performed in any order or in parallel. Deviating from the present non-limiting example, steps S250 and/or S260A can also be omitted, for example if the subsequent step (S300A) is performed using a blunt ligation or if the DNA fragment 10 and/or the hairpin oligonucleotides 30a, 30b are already provided with the desired overhang sequences.

The first hairpin oligonucleotide 30a and the second hairpin oligonucleotide 30b may be different or identical to each other. In the present non-limiting example, the first hairpin oligonucleotide 30a and the second hairpin oligonucleotide 30b are identical to each other.

Figure 9A shows the structure of the first and second hairpin oligonucleotides 30a and 30b. The first and second hairpin oligonucleotides 30a and 30b are respectively single-stranded oligonucleotides having a hairpin structure. A hairpin structure is a trunk-loop structure with a double-stranded stem 32 and a short single-stranded loop 33. The hairpin oligonucleotide has two complementary sections 34 and 34' which form the double-stranded stem 32 and which include an unpaired intermediate section forming the single-stranded loop 33.

Figure 4 shows the subsequent step (S300A) and the subsequent steps (S500A) and (S600A) performed together.

In step (S300A), the 5' end of the first strand 20 is joined to the 3' end of the first hairpin oligonucleotide 30a and the 3' end of the second strand 20' is joined to a 5' end of the first hairpin oligonucleotide 30a, as well as the 3' end of the first strand 20 is joined to a 5' end of the second hairpin oligonucleotide 30b and the 5' end of the second strand 20' is joined to the 3' end of the second hairpin oligonucleotide 30b. The first hairpin oligonucleotide 30a and the second hairpin oligonucleotide 30b each provide a single-stranded section of the resulting ligated product 40 by their loop 33. The correct connection of the two ends of DNA fragment 10 to the two hairpin oligonucleotides 30a and 30b is facilitated by the fact that there is a base pairing between the overhang sequences 21 of the DNA fragment 10 and the overhang sequences 31 of the first and second hairpin oligonucleotides 30a and 30b.

Step (S300A) physically connects the first strand 20 and the second strand 20' of DNA fragment 10 at the two ends of DNA fragment 10. The resulting ligated product 40 is a circular DNA molecule that acts as a circular DNA template for rolling circle amplification (RCA) in step (S500A). In this way, a so-called Rolling Circle library for sequencing can be produced. The connected product 40 has a dumbbell structure.

It is possible to perform a selection of a correctly ligated product 40 obtained in step (S300A) using an exonuclease treatment in a further step (S400A). Exonuclease breaks down DNA molecules with a free 5' end or a free 3' end. Since a product 40 correctly joined in step (S300) has neither a free 5' end nor a free 3' end, it is protected from degradation by the exonuclease. In the present non-limiting example, the procedure is continued without the selection step (S400A).

The connected product 40 obtained in step (S300A) is amplified by RCA in step (S500A). In the present non-limiting example, step (S500A) and step (S600A) are performed together, *i.e.* the RCA is performed using a first group of redox-modified nucleotides 50 and a second group of redox-modified nucleotides 55. Each redox-modified nucleotide 50 in the first group has a first redox species with an initial oxidation or reduction potential, and each redox-modified nucleotide 55 in the second group has a first redox species with a first oxidation or reduction potential. In the present non-limiting example, the first group consists of redox-modified nucleotides 50 of the type deoxyuridine triphosphate (dUTP), where the first redox species is the methylene blue derivative ATTO MB2 and the redox-modified nucleotide 50 is also referred to as MB-dUTP (see Fig. 2A). The second group consists of redox-modified nucleotides 55 of the species deoxycytidine triphosphate (dCTP), where the second redox species is the ferrocene derivative FcN-C2 and the redox-modified nucleotide 55 is also referred to as FcN-C2-dCTP (see Fig. 2B). In addition, non-redox-modified nucleotides of the species deoxyadenosine triphosphate (dATP) and deoxyguanosine triphosphate (dGTP) (not shown), a DNA polymerase with strand displacement activity, namely the vent (exo-) DNA polymerase from New England Biolabs, Inc. (New England Biolabs, Inc., Ipswich, MA, USA) (not shown), and a type of primer 60 are used for RCA. RCA is performed in a suitable buffer that is compatible with DNA polymerase. The primer 60 is chosen to bind to a primer binding site 60' (see Fig. 9A) provided by the single-strand section (*i.e.* loop 33) of the first and second hairpin oligonucleotides 30a and 30b in the ligated product 40. The starting point 70 of the RCA is marked with an arrow in Fig. 4. The DNA polymerase continuously attaches individual nucleotides to the primer 60. In this way, RCA results in two long concatemeric single-stranded DNA molecules, each of which has a large number of copies, each complementary to the circular DNA template. These single-stranded DNA molecules are amplicons 80, which have the redox-modified nucleotides 50, 55 (redox-labeled amplicons 80), and which are sequenced in a further step (S800A). Each of the single-stranded amplicons 80 has multiple copies that are complementary to the sequence of the first strand 20 and multiple copies that are complementary to the sequence of the second strand 21', the copies being the sequence of the first strand 20 are complementary, alternate with the copies that are complementary to the sequence of the second strand 20'. A sequence that is complementary to the sequence of the first hairpin oligonucleotide 30a or the second hairpin oligonucleotide 30b is arranged between the individual copies.

Alternatively, it is possible to perform steps (S500A) and (S600A) separately one after the other, *i.e.* the ligated product 40 obtained in step (S300A) is first amplified in step (S500A) by RCA using only non-redox-modified nucleotides. Then the amplicons obtained in step (S500A) are amplified in step (S600A) by primer extension using the first group of redox-modified nucleotides 50 and the second group of redox-modified nucleotides 55.

The primer 60 has a label 61 which allows a selection of the amplicons 80 obtained in the jointly performed steps (S500A) and (S600A), which have the redox-modified nucleotides 50, 55 (step S700A). In the present non-limiting example, the label is biotin, *i.e.* a biotinylated primer 60 is used. As a result, the amplicons 80, which have the redox-modified nucleotides 50, 55, have a biotin molecule. The circular DNA matrix, on the other hand, does not have a biotin molecule.

In a further step (S700A), amplicons 80 are selected by streptavidin-purification using streptavidin-coupled beads using the specific interaction between streptavidin and biotin. The amplicons 80 selected in step (S700A) are used instead of the amplicons 80 obtained in step (S600A) in the subsequent step (S800A).

Deviating from the present non-limiting example, step (S700A) can also be omitted.

In a further step (S800A), the sequence of the DNA strand is determined by sequencing the amplicons 80 selected in step (S700A) using an electrochemical sequencing method. If step (S700A) is omitted, in contrast to this non-limiting example, the sequence of the DNA strand is determined by sequencing the amplicons 80 obtained in step (S600A). In the electrochemical sequencing method, the redox-modified nucleotides 50, 55 of the amplicons 80 are oxidized or reduced along each amplicon in turn. Oxidizing or reducing produces an electrochemical signal specific to a particular type of redox species so the electrochemical signal identifies the redox species of redox-modified nucleotide 50, 55. Each redox species is specific to a particular type of nucleotide so that the redox species can be used to infer which type of nucleotide is incorporated at a certain position in the redox-labeled amplicon. This is the basis for the sequencing of the redox-labeled amplicons 80. In the present non-limiting example, the identification of MB-dUTP is based on the base thymine and the identification of FcN-C2-dCTP is based on the base cytosine in the sequence of the redox-labeled amplicon. A gap in the electrochemical signal is detected at the points where the sequence of the redox-labeled amplicon contains the base adenine or the base guanine.

Sequencing identifies the sequence of copies that are complementary to the sequence of the first strand 20 as well as the sequence of copies that are complementary to the sequence of the second strand 20'. Based on the complementarity of the copies, the sequence of the first strand 20 and the sequence of the second strand 20' can be identified. Due to the complementarity of the first and second strands 20 and 20', the complete sequence of the first strand 20 and the second strand 20' can then be determined by merging the information obtained. Thus, the sequence of the initial DNA strand, which corresponds to the first strand 20 in the present non-limiting example, can be determined from the sequence of redox-labeled amplicons 80.

The electrochemical sequencing method used is sequencing by electronic tunneling, which is known from WO 2019/086305 A1. The electrochemical sequencing process involves the following steps: (a) providing a redox-labeled amplicon; (b) applying a voltage to at least one edge electrode; (c) passing the amplicon over at least one edge electrode; (d) oxidizing or reducing each redox-modified nucleotide using at least one edge electrode, while passing the respective redox-modified nucleotide over at least one edge electrode, wherein oxidizing or reducing produces an electrochemical signal involving a change in current; (e) identifying each redox-modified nucleotide based on the electrochemical signal; and (f) determining a sequence of the amplicon.

Thus, the sequence of the DNA strand is determined by sequencing the redox-labeled amplicons 80. The sequence of the DNA strand is determined from the sequence of redox-labeled amplicons 80. The sequencing of the redox-labeled amplicons 80 does not take place during the production of the same, but after their production.

Since each of the single-stranded amplicons has 80 multiple copies that are complementary to the sequence of the first strand 20 and multiple copies that are complementary to the sequence of the second strand 20', the Rolling Circle method allows not only the complete reconstruction of the sequence of the initial DNA strand but also the simultaneous determination of the consensus sequence of the DNA strand. This increases the accuracy of sequence determination.

A process flow of the second method for sequencing of a DNA strand will now be explained with reference to Figs. 5, 6, and 9B. The second method can also be referred to as the UMI method.

In a first step (S200), the DNA strand to be sequenced is provided as a strand of a double-stranded DNA fragment 10. For details on double-stranded DNA fragment 10, please refer to the examples of the Rolling Circle method.

In a further step (S250), a dA-tailing of the first strand 20 is performed, in which a dATP is attached to the 3' end of the first strand 20, and a dA-tailing of the second strand 20' is performed, in which a dATP is attached to the 3' end of the second strand 20'. In this way, 10 overhang sequences 21, each consisting of a single deoxyadenosine, are produced at the two ends of the DNA fragment.

In a further step (S260B), a dT-tailing of a first UMI oligonucleotide 130 is performed, in which a dTTP is attached to a 3' end of the first UMI oligonucleotide 130, and a dT-tailing of a second UMI oligonucleotide 135 is performed, in which a dTTP is attached to a 3' end of the second UMI oligonucleotide 135. In this way, overhang sequences 31, each consisting of a single deoxythymidine, are produced at the 3' end of the first UMI oligonucleotide 130 and the 3' end of the second UMI oligonucleotide 135.

Steps (S250) and (S260B) can be performed in any order or in parallel. Deviating from the present non-limiting example, steps (S250) and/or (S260B) can also be omitted, for example if the subsequent step (S300B) is performed by a blunt ligation or if the DNA fragment 10 and/or the UMI oligonucleotides 130, 135 are already provided with the desired overhang sequences.

Fig. 9B shows the structure of the first and second UMI oligonucleotides 130 and 135. The first and second UMI oligonucleotides 130 and 135 are respectively a two-stranded, Y-shaped oligonucleotide with a paired end portion 132 and an unpaired end portion 133, wherein the paired end portion 132 has a unique molecular identification sequence (UMI sequence) 134, 136 arranged terminally, with UMI sequence 134 of the first UMI oligonucleotide 130 distinct from UMI sequence 136 of the second UMI oligonucleotide 135. The term "UMI" stands for unique molecular identifier. The UMI sequence 134, 136 consists of a predetermined unique random sequence of nucleotides. The paired end portion 132 has further double-stranded section adjacent to the section which has the UMI sequence 134, 136 and the complementary sequence 134', 136'. This further double-stranded section, the strands of which are marked 137, 137' in Fig. 9B, is arranged between the section containing the UMI sequence 134, 136 and the complementary sequence 134', 136', and the unpaired end portion 133 of the UMI oligonucleotide 130, 135. In the unpaired end portion 133, also known as the asymmetric flanking site, the sequences of the two strands of the UMI oligonucleotide 130, 135 are not complementary to each other. The unpaired end portion 133 of the first and second UMI oligonucleotides 130 and 135 may be different or identical to each other. In the present embodiment, the unpaired end portion 133 of the first UMI oligonucleotide 130 and the unpaired end portion 133 of the second UMI oligonucleotide 135 are identical.

Fig. 6 shows the following step (S300B) and the subsequent steps (S500B) and (S600B) successively.

In step (S300B), the 5' end of the first strand 20 and the 3' end of the second strand 20' are joined to the first UMI oligonucleotide 130 and the 3' end of the first strand 20 and the 5' end of the second strand 20' are joined to the second UMI oligonucleotide 135, with the paired end portion 132 of the first and second UMI oligonucleotides 130 and 135 connected to the first strand 20 and the second strand 20' respectively becomes. By joining, the first strand 20 is connected to the UMI sequence 134 of the first UMI oligonucleotide 130 and the second strand 20' to the UMI sequence 136 of the second UMI oligonucleotide 135. The correct connection of the two ends of DNA fragment 10 to the two UMI oligonucleotides 130 and 135 is facilitated by the fact that there is a base pairing between the overhang sequences 21 of the DNA fragment 10 and the overhang sequences 31 of the first and second UMI oligonucleotides 130 and 135.

In step (S300B), the first strand 20 and the second strand 20' of DNA fragment 10 remain only non-physically connected to each other, but the first strand 20 and the second strand 20' can be clearly identified and assigned to each other by the UMI oligonucleotides 130, 135 after sequencing.

Step (S300B) creates a ligated product 140 which acts as a DNA template for polymerase chain reaction (PCR) in step (S500B). In this way, a so-called UMI library can be produced for sequencing.

The ligated product 140 obtained in step (S300B) is amplified by PCR in step (S500B). PCR is performed using only non-redox-modified nucleotides (dNTPs) of the species dATP, dCTP, dGTP, and dTTP (not shown). PCR also uses a DNA polymerase (not shown) and a type of primer 141. PCR is performed in a suitable buffer compatible with DNA polymerase. The primer 141 binds to a primer binding site 141' (see Fig.9B), which is provided by the first and second UMI oligonucleotides 130 and 135 respectively at the 3' end of the unpaired end portion 133. The DNA polymerase continuously attaches individual nucleotides to the primers 141. In this way, PCR results in multiple amplicons 145 of the bonded product 140 with the primer-binding site 141' labeling the beginning and end of each amplicon 145 respectively, and amplicons 145 being double-stranded along their entire length. The multiple amplicons 145 can also be referred to as the so-called "UMI clone."

The amplicons 145 obtained in step (S500B) are amplified in step (S600B) using primer extension. Amplification is performed using a first group of redox-modified nucleotides 50 and a second group of redox-modified nucleotides 55. Each redox-modified nucleotide 50 in the first group has a first redox species with a first oxidation or reduction potential, and each redox-modified nucleotide 55 in the second group has a second redox species with a second oxidation or reduction potential.

In the present non-limiting example, the first group consists of redox-modified nucleotides 50 of the type deoxyuridine triphosphate (dUTP), where the first redox species is the methylene blue derivative ATTO MB2 and the redox-modified nucleotide 50 is also referred to as MB-dUTP (see Fig. 2A). The second group consists of redox-modified nucleotides 55 of the species deoxycytidine triphosphate (dCTP), where the second redox species is the ferrocene derivative FcN-C2 and the redox-modified nucleotide 55 is also referred to as FcN-C2-dCTP (see Fig. 2B). In addition, non-redox-modified nucleotides of the species dATP and dGTP (not shown), a DNA polymerase, namely the vent (exo-) DNA polymerase from New England Biolabs, Inc. (New England Biolabs, Inc., Ipswich, MA, USA) (not shown), and two different types of primers 162, 163 are used for amplification. Amplification is carried out using primer extension. The DNA matrices for the primer extension are amplicons 145 obtained in step (S500B). Primer extension is performed in a suitable buffer that is compatible with DNA polymerase. The primers 162, 163 are chosen in such a way that they can bind to the amplicons 145. In the present non-limiting example, the first primer 162 binds to a primer binding site that corresponds to the primer binding site 141' as already used for PCR in step (S500B). This primer-binding site is arranged by PCR at one end of amplicons 145. The second primer 163 binds to a primer binding site arranged by PCR at the other end of amplicon 145 and which corresponds to the 5' end of the unpaired end portion 133 of the first and second UMI oligonucleotides 130 and 135. The starting point 170 of the primer extension is marked with an arrow in Fig. 6. The DNA polymerase continuously attaches individual nucleotides to primers 162, 163. In this way, the primer extension leads to single-stranded or double-stranded DNA molecules containing the redox-modified nucleotides 50, 55 (redox-labeled amplicons 180), which are sequenced in a further step (S800B). Each redox-labeled single strand of amplicon 180 contains either one copy of the first strand 20 of DNA fragment 10 or one copy of the second strand 20' of DNA fragment 10. The redox-labeled single strands, which comprise one copy of the first strand 20 of DNA fragment 10, have the UMI sequence 134 of the first UMI oligonucleotide 130, and the sequence 136' which is complementary to the UMI sequence 136 of the second UMI oligonucleotide 135. The redox-labeled single strands, which comprise one copy of the second strand 20' of DNA fragment 10, have the UMI sequence 136 of the second UMI oligonucleotide 135, as well as the sequence 134' complementary to the UMI sequence 134 of the first UMI oligonucleotide 130. As a result, the first strand 20 and the second strand 20' of DNA fragment 10 can be clearly identified and assigned to each other after sequencing.

The primers 162, 163 each have a label 61, which allows a selection of the amplicons 180 obtained in step (S600B), which have the redox-modified nucleotides 50, 55 (step S700B). In the present non-limiting example, the label is biotin, i.e. biotinylated primers 162, 163 are used. As a result, the amplicons 180, which have the redox-modified nucleotides 50, 55, have a biotin molecule. The amplicons 145 used as DNA matrices, on the other hand, do not contain a biotin molecule.

In a further step (S700B), the amplicons 180 are selected by a streptavidin purification using streptavidin-coupled beads using the specific interaction between streptavidin and biotin. The amplicons 180 selected in step (S700B) are used instead of the amplicons 180 obtained in step (S600B) in the subsequent step (S800B).

Deviating from the present non-limiting example, step S700B can also be omitted.

In a further step (S800B), the sequence of the DNA strand is determined by sequencing the amplicons 180 selected in step (S700B) using an electrochemical sequencing method. If step (S700B) is omitted from the present embodiment, the sequence of the DNA strand is determined by sequencing the amplicons 180 obtained in step (S600B). Since only two types of redox-modified nucleotides 50, 55 were used in the present non-limiting example, the redox-modified nucleotides 50, 55 must be identified in the copies of the first strand 20 as well as in the copies of the second strand 20' during sequencing. Due to the complementarity of the first and second strands 20 and 20', the complete sequence of the first strand 20 and the second strand 20' can then be determined by merging the information obtained. Thus, the sequence of the initial DNA strand, which corresponds to the first strand 20 in the present non-limiting example, can be determined from the sequence of redox-labeled amplicons 180.

For details on the electrochemical sequencing method, please refer to the example of the Rolling Circle method.

The sequence of the DNA strand is determined by sequencing the redox-labeled amplicons 180. The sequence of the DNA strand is determined from the sequence of redox-labeled amplicons 180. The sequencing of the redox-labeled amplicons 180 does not take place during the production of the same, but after their production.

The UMI method allows a complete reconstruction of the sequence of the initial DNA strand. The method also enables the determination of the consensus sequence of the DNA strand by comparing the results of several readouts showing an identical UMI sequence 134, 136. This increases the accuracy of sequence determination.

With reference to Figs. 7, 8, and 9C, a procedure sequence of a third method for sequencing a DNA strand is now explained. The third method can also be referred to as the 2D method.

In a first step (S200), the DNA strand to be sequenced is provided as a strand of a double-stranded DNA fragment 10. For details on double-stranded DNA fragment 10, please refer to the explanations of the Rolling Circle method.

In a further step (S250), a dA-tailing of the first strand 20 is performed, in which a dATP is attached to the 3' end of the first strand 20, and a dA-tailing of the second strand 20' is performed, in which a dATP is attached to the 3' end of the second strand 20'. In this way, 10 overhang sequences 21, each consisting of a single deoxyadenosine, are produced at the two ends of the DNA fragment.

In a further step (S260C), a dT-tailing of a first oligonucleotide 230 is performed, in which a dTTP is attached to the 3' end of the first oligonucleotide 230, and a dT-tailing of a second oligonucleotide 235 is performed, in which a dTTP is attached to the 3' end of the second oligonucleotide 235. In this way, at the 3' end of the first oligonucleotide 230 and the 3' end of the second oligonucleotide 235, overhang sequences 31, each consisting of a single deoxythymidine, are produced.

Steps (S250) and (S260C) can be performed in any order or in parallel. Deviating from the present non-limiting example, steps (S250) and/or (S260C) can also be omitted, for example if the subsequent step (S300C) is performed by a blunt ligation or if the DNA fragment 10 and/or the oligonucleotides 230, 235 are already provided with the desired overhang sequences.

Fig. 9C shows the structure of the first and second oligonucleotides 230 and 235. In the present non-limiting example, the first oligonucleotide 230 is a hairpin oligonucleotide and the second oligonucleotide 235 is a Y-shaped oligonucleotide. Alternatively, the first oligonucleotide may be a Y-shaped oligonucleotide and the second oligonucleotide may be a hairpin oligonucleotide.

The hairpin oligonucleotide (first oligonucleotide 230) is a single-stranded oligonucleotide that has a hairpin structure. A hairpin structure is a trunk-loop structure with a double-stranded stem 232 and a short single-stranded loop 233. The hairpin oligonucleotide has two complementary sections 234 and 234' which form the double-stranded stem 232 and which include an unpaired intermediate section, the unpaired intermediate section forming the single-stranded loop 233.

The Y-shaped oligonucleotide (second oligonucleotide 235) is a two-stranded oligonucleotide with a paired end portion 238 and an unpaired end portion 239, wherein the paired end portion 238 in the present non-limiting example has a unique molecular identification sequence (UMI sequence) 236 arranged on a strand. The UMI sequence 236 consists of a predetermined, unique random sequence of nucleotides. The paired end portion 238 has another double-stranded interval adjacent to the section that has the UMI sequence 236 and the complementary sequence 236'. This further double-stranded interval, the strands of which are marked 237, 237' in Fig. 9C, is situated between the interval containing the UMI sequence 236 and the complementary sequence 236' and the unpaired end portion 239 of the Y-shaped oligonucleotide. In the unpaired end portion 239, also known as the asymmetric flanking site, the sequences of the two strands of the Y-shaped oligonucleotide are not complementary to each other.

Alternatively, the hairpin oligonucleotide may have a UMI sequence that is terminally arranged. In this case, the UMI sequence and the complementary sequence form the end portion of the stem of the hairpin oligonucleotide. In another alternative, both the Y-shaped oligonucleotide and the hairpin oligonucleotide may have a UMI sequence, each of which is terminally arranged.

Fig. 8 shows the subsequent steps (S300C) and (S600C).

In step (S300C), the 5' end of the first strand 20 and the 3' end of the second strand 20' are joined to the first oligonucleotide 230, and the 3' end of the first strand 20 and the 5' end of the second strand 20' are joined to the second oligonucleotide 235. The paired end portion 238 of the second oligonucleotide 235 is joined to the first strand 20 and the second strand 20'. The first oligonucleotide 230 provides a single-stranded portion of the resulting ligated product 240 by its loop 233. By joining, the second strand 20' is joined to the UMI sequence 236 of the second oligonucleotide 235. The correct connection of the two ends of DNA fragment 10 to the two oligonucleotides 230 and 235 is facilitated by the fact that there is a base pairing between the overhang sequences 21 of the DNA fragment 10 and the overhang sequences 31 of the first and second oligonucleotides 230 and 235.

In step (S300C), the first strand 20 and the second strand 20' of the DNA fragment 10 are physically connected to each other via the hairpin oligonucleotide at one end. The resulting ligated product 240 acts as a DNA template for the primer extension in step (S600C). In this way, a so-called "two-dimensional library" ("2D library") can be produced for sequencing.

It is possible, in a further step (S400C), to perform a selection of a correctly ligated product 240 obtained in step (S300C) using a first scavenger oligonucleotide binding to a specific unique segment of the first oligonucleotide 230 and using a second capture oligonucleotide binding to a specific unique segment of the second oligonucleotide 235. For this purpose, the first scavenger oligonucleotide can be used in a first stage. For example, the first catcher oligonucleotide can be a biotinylated first catcher oligonucleotide. Any product associated with the first oligonucleotide 230 can then be selected by streptavidin purification. In a second stage of the selection process, the selected products from the first stage and the second scavenger oligonucleotide are used. For example, the second catcher oligonucleotide can be a biotinylated second catcher oligonucleotide. Each selected product from the first stage, which is associated with the second oligonucleotide 235, can then be selected by streptavidine purification. Thus, each selected product from the second stage is associated with both the first oligonucleotide 230 and the second oligonucleotide 235, *i.e.* each selected product from the second stage is a correctly ligated product 240.

It is also possible to amplify the compound product selected in step (S400C) by polymerase chain reaction (PCR) in a further step (S450C). In this way, multiple amplicons of the selected product are obtained, with the amplicons being double-stranded over their entire length. The amplicons, like the bonded product 240 obtained in step (S300C), can be amplified in step (S600C) by primer extension using the first group of redox-modified nucleotides 50 and the second group of redox-modified nucleotides 55.

In the present non-limiting example, the process is continued without the selection step (S400C) and, accordingly, without the enrichment step (S450C).

The ligated product 240 obtained in step (S300C) is amplified in step (S600C) by primer extension. Primer extension is performed using a first group of redox-modified nucleotides 50 and a second group of redox-modified nucleotides 55, wherein each redox-modified nucleotide 50 in the first group has a first redox species with a first oxidation or reduction potential and each redox-modified nucleotide 55 in the second group has a second redox species with a second oxidation or reduction potential. In the present non-limiting example, the first group consists of redox-modified nucleotides 50 of the type deoxyuridine triphosphate (dUTP), where the first redox species is the methylene blue derivative ATTO MB2, and the redox-modified nucleotide 50 is also referred to as MB-dUTP (see Fig. 2A). The second group consists of redox-modified nucleotides 55 of the species deoxycytidine triphosphate (dCTP), where the second redox species is the ferrocene derivative FcN-C2, and the redox-modified nucleotide 55 is also referred to as FcN-C2-dCTP (see Fig. 2B). In addition, non-redox-modified nucleotides of the species dATP and dGTP (not shown), a DNA polymerase, namely the vent (exo-) DNA polymerase from New England Biolabs, Inc. (New England Biolabs, Inc., Ipswich, MA, USA) (not shown), and a type of primer 260 are used for amplification. The DNA matrix for the primer extension is the ligated product 240 obtained in step (S300C). Primer extension is performed in a suitable buffer that is compatible with DNA polymerase. The primer 260 is chosen to bind to a primer binding site 260' (see Fig. 9C), which is provided by the second oligonucleotide 235 at the 3' end of the unpaired final segment 239. The starting point 270 of the primer extension is marked with an arrow in Fig. 8. The DNA polymerase continuously attaches individual nucleotides to the primer 260. In this way, the primer extension leads to single-stranded or double-stranded DNA molecules containing the redox-modified nucleotides 50, 55 (redox-labeled amplicons 280), which are sequenced in a further step (S800C). Each redox-labeled single strand of amplicon 280 comprises a single copy complementary to the sequence of the first strand 20 of DNA fragment 10 and a single copy complementary to the sequence of the second strand 20' of DNA fragment 10. In addition, each redox-labeled single strand of amplicons 280 has sequence 236', which is complementary to the UMI sequence 236 of the second oligonucleotide 235.

The primer 260 has a label 61, which allows a selection of the amplicons 280 obtained in step (S600C), which have the redox-modified nucleotides 50, 55 (step S700C). In the present non-limiting example, the labeling is biotin, *i.e.* a biotinylated primer 260 is used. As a result, amplicons 280, which have the redox-modified nucleotides 50, 55, have a biotin molecule. On the other hand, the associated product 240 used as a DNA template does not contain a biotin molecule.

In a further step (S700C), the amplicons 280 are selected by a streptavidin-purification using streptavidin-coupled beads using the specific interaction between streptavidin and biotin. The amplicons 280 selected in step (S700C) are used instead of the amplicons 280 obtained in step (S600C) in the subsequent step (S800C).

Deviating from the present non-limiting example, step (S700C) can also be omitted.

In a further step (S800C), the sequence of the DNA strand is determined by sequencing the amplicons 280 selected in step (S700C) using an electrochemical sequencing method. If step (S700C) is omitted in a way that differs from the non-limiting example, the sequence of the DNA strand is determined by sequencing the amplicons 280 obtained in step (S600C). Since only two types of redox-modified nucleotides 50, 55 were used in the present non-limiting example, the redox-modified nucleotides 50, 55 need to be identified during sequencing both in the copies that are complementary to the sequence of the first strand 20 and in the copies that are complementary to the sequence of the second strand 20'. Based on the complementarity of the copies, the sequence of the first strand 20 and the sequence of the second strand 20' can be identified. Due to the complementarity of the first and second strands 20 and 20', the complete sequence of the first strand 20 and the second strand 20' can then be determined by merging the information obtained. Thus, the sequence of the initial DNA strand, which corresponds to the first strand 20 in the present non-limiting example, can be determined from the sequence of the redox-labeled amplicons 280.

For details on the electrochemical sequencing method, please refer to the explanations of the Rolling Circle method.

The sequence of the DNA strand is determined by sequencing the redox-labeled amplicons 280. The sequence of the DNA strand is determined from the sequence of redox-labeled amplicons 280. The sequencing of the redox-labeled amplicons 280 does not take place during the production of the same, but after their production.

The 2D method enables a complete reconstruction of the sequence of the initial DNA strand. In addition, the 2D method requires less computing power to reconstruct a complete sequence from a single molecule compared to the use of two or more molecules such as those required by the UMI method.

In the present non-limiting example, the method also enables the determination of the consensus sequence of the DNA strand by comparing the results of several readout processes showing the sequence 236' which is complementary to the UMI sequence 236. This increases the accuracy of sequence determination.

In contrast to the present non-limiting examples, the methods for sequencing a DNA strand may also include the upstream steps (S100) and (S150). In step (S100), a double-stranded DNA is fragmented into double-stranded DNA fragments with a first strand that has a 5' end and a 3' end and a second strand that has a 5' end and a 3' end. In step (S150), a final DNA repair of the DNA fragments obtained in step (S100) is performed. In this process, sticky ends of the DNA fragments obtained in step (S100) are repaired and blunt ends are created. Steps (S100) and (S150) result in double-stranded DNA fragments, each of which can be provided in step (S200) of the method. By determining the sequence of several of the DNA fragments, it is possible to determine the sequence of double-stranded DNA, or at least a longer section of double-stranded DNA.

In contrast to the present non-limiting examples, the methods for sequencing a DNA strand can also be used for sequencing an RNA strand by transcribing the RNA into complementary DNA (cDNA) by reverse transcription in a step upstream of the procedure.

While exemplary embodiments are described above, it is not intended that these embodiments describe all possible forms of the invention. Rather, the words used in the specification are words of description rather than limitation, and it is understood that various changes may be made without departing from the spirit and scope of the invention. Additionally, the features of various implementing embodiments may be combined to form further embodiments of the invention.

## Claims

1. A method for sequencing of a DNA strand, the method comprising:
(S200) providing a double-stranded DNA fragment with a first strand having a 5' end and a 3' end and a second strand having a 5' end and a 3' end, the first and the second strands being complementary to each other, the DNA strand to be sequenced corresponding to the first strand or the second strand;
(S300A) forming a ligated product by connecting:
the 5' end of the first strand to a 3' end of a first hairpin oligonucleotide,
the 3' end of the second strand to a 5' end of the first hairpin oligonucleotide,
the 3' end of the first strand to a 5' end of a second hairpin oligonucleotide, and
the 5' end of the second strand to a 3' end of the second hairpin oligonucleotide,
the first hairpin oligonucleotide and the second hairpin oligonucleotide each providing a single-stranded section of the ligated product;
(S500A) amplifying the ligated product obtained in step (S300A) by rolling circle amplification (RCA) to produce amplicons of the ligated product;
(S600A) amplifying the ligated product obtained in step (S300A) or the amplicons of the ligated product obtained in step (S500A) by primer extension using a first group of redox-modified nucleotides and a second group of redox-modified nucleotides, each redox-modified nucleotide in the first group having a first redox species with a first oxidation or reduction potential and each redox-modified nucleotide in the second group having a second redox species with a second oxidation or reduction potential, the first group consisting of deoxyadenosine triphosphate (dATP), deoxythymidine triphosphate (dTTP), or deoxyuridine triphosphate (dUTP), the second group consisting of deoxycytidine triphosphate (dCTP) or deoxyguanosine triphosphate (dGTP); and
(S800A) determining the sequence of the DNA strand by sequencing the amplicons obtained in step (S600A) by electrochemical sequencing.

2. The method of claim 1, further comprising (S400A) selecting a correctly ligated product obtained in step (S300A) by an exonuclease treatment.

3. The method of claim 1, further comprising (S700A) selecting only the amplicons having the redox modified nucleotides obtained in the step (S600A) for the step (S800A).

4. The method of claim 3, wherein the (S700B) selecting is by streptavidine purification.

5. The method of claim 1, further comprising (S250) forming overhang sequences in the double-stranded DNA fragment by dA-tailing of the first and second strands and (S260A) forming overhang sequences by dT-tailing of the first and second hairpin oligonucleotides.

6. The method of claim 1, further comprising (S100) fragmenting the double-stranded DNA into double-stranded DNA fragments with the first strand having the 5' end and a 3' end and the second strand having the 5' end and a 3' end, and (S150) performing DNA end repair of the DNA fragments obtained in the step (S100).

7. A method for sequencing of a DNA strand, the method comprising:
(S200) providing a double-stranded DNA fragment with a first strand having a 5' end and a 3' end and a second strand having a 5' end and a 3' end, the first and the second strands being complementary to each other, the DNA strand to be sequenced corresponding to the first strand or the second strand;
(S300B) forming a ligated product by connecting:
the 5' end of the first strand and the 3' end of the second strand to a first UMI oligonucleotide, and
the 3' end of the first strand and the 5' end of the second strand to a second UMI oligonucleotide,
the first UMI oligonucleotide and the second UMI oligonucleotide each being two-stranded, Y-shaped oligonucleotides having a paired end portion and an unpaired end portion, the paired end portions being connected to the first strand and the second strand, respectively, the paired end portion on each strand having a unique molecular identification sequence (UMI sequence) arranged terminally, the UMI sequence of the first UMI oligonucleotide being different from the UMI sequence of the second UMI oligonucleotide, the first strand being connected to the UMI sequence of the first UMI oligonucleotide and the second strand being connected to the UMI sequence of the second UMI oligonucleotide;
(S500B) amplifying the ligated product obtained in step (S300B) using polymerase chain reaction (PCR) to generate amplicons;
(S600B) amplifying the amplicons obtained in step (S500B) by primer extension using a first group of redox-modified nucleotides and a second group of redox-modified nucleotides, each redox-modified nucleotide in the first group having a first redox species with a first oxidation or reduction potential and each redox-modified nucleotide in the second group having a second redox species with a second oxidation or reduction potential, the first group consisting of deoxyadenosine triphosphate (dATP), deoxythymidine triphosphate (dTTP), or deoxyuridine triphosphate (dUTP), the second group consisting of deoxycytidine triphosphate (dCTP) or deoxyguanosine triphosphate (dGTP); and
(S800B) determining the sequence of the DNA strand by sequencing the amplicons obtained in step (S600B) by electrochemical sequencing.

8. The method of claim 7, further comprising (S700B) selecting only the amplicons having the redox modified nucleotides for the step (S800B).

9. The method of claim 8, wherein the (S700B) selecting further comprises removing DNA molecules lacking the redox modified nucleotides.

10. The method of claim 7, further comprising (S250) forming overhang sequences in the double-stranded DNA fragment by dA-tailing of the first and second strands and (S260B) forming overhang sequences by dT-tailing of the first UMI oligonucleotide and the second UMI oligonucleotide.

11. The method of claim 7, further comprising (S100) fragmenting the double-stranded DNA into double-stranded DNA fragments with the first strand having the 5' end and a 3' end and the second strand having the 5' end and a 3' end, and (S150) performing DNA end repair of the DNA fragments obtained in the step (S100).

12. A method for sequencing of a DNA strand, the method comprising:
(S200) providing a double-stranded DNA fragment with a first strand having a 5' end and a 3' end and a second strand having a 5' end and a 3' end, the first and the second strands being complementary to each other, the DNA strand to be sequenced corresponding to the first strand or the second strand;
(S300C) forming a ligated product by connecting:
the 5' end of the first strand and the 3' end of the second strand to a first oligonucleotide, and
the 3' end of the first strand and the 5' end of the second strand to a second oligonucleotide,
one of the first and second oligonucleotides being a hairpin oligonucleotide and the other of the first and second oligonucleotides being a Y-shaped oligonucleotide; the hairpin oligonucleotide providing a single-stranded portion of the ligated product, the Y-shaped oligonucleotide being a two-stranded oligonucleotide with a paired end portion and an unpaired end portion, the paired end portion being connected to the first strand and the second strand,
(S600C) forming amplicons by amplifying the ligated product obtained in step (S300C) by primer extension using a first group of redox-modified nucleotides and a second group of redox-modified nucleotides, each redox-modified nucleotide in the first group having a first redox species with a first oxidation or reduction potential and each redox-modified nucleotide in the second group having a second redox species with a second oxidation or reduction potential, the first group consisting of deoxyadenosine triphosphate (dATP), deoxythymidine triphosphate (dTTP), or deoxyuridine triphosphate (dUTP), the second group consisting of deoxycytidine triphosphate (dCTP) or deoxyguanosine triphosphate (dGTP); and
(S800C) determining the sequence of the DNA strand by sequencing the amplicons obtained in step (S600C) by electrochemical sequencing.

13. The method of claim 12, wherein the paired end portion of the Y-shaped oligonucleotide on one strand has a UMI sequence arranged terminally.

14. The method of claim 12, wherein the hairpin oligonucleotide has a UMI sequence arranged terminally.

15. The method of claim 12, further comprising (S700C) selecting only the amplicons having the redox modified nucleotides obtained in step (S600C) for the step (S800C).

16. The method of claim 15, wherein the (S700C) selecting is by streptavidine purification.

17. The method of claim 12, further comprising (S400C) selecting a correctly ligated product obtained in step (S300A) by a first capture oligonucleotide binding to a specific unique portion of the first oligonucleotide and by a second capture oligonucleotide binding to a specific unique portion of the second oligonucleotide.

18. The method of claim 17, further comprising (S450C) amplifying the ligated product selected in step (S400C) using polymerase chain reaction (PCR).

19. The method of claim 12, further comprising (S250) forming overhang sequences in the double-stranded DNA fragment by dA-tailing of the first and second strands and (S260C) forming overhang sequences by dT-tailing of the first oligonucleotide and the second oligonucleotide.

20. The method of claim 12, further comprising (S100) fragmenting the double-stranded DNA into double-stranded DNA fragments with the first strand having the 5' end and a 3' end and the second strand having the 5' end and a 3' end, and (S150) performing DNA end repair of the DNA fragments obtained in the step (S100).
